# EUROPEAN PATENT SPECIFICATION

(11) **EP 0 811 593 B1**
(45) Date of publication and mention of the grant of the patent: **24.07.2002**
(21) Application number: 97303657.7
(22) Date of filing: 29.05.1997
(51) Int. Cl.: C07C 25/24, C07C 43/29, C07C 255/34, C07C 255/38, A01N 31/14

(54) **1,4-diaryl-2-fluoro-2-butene insecticidal and acaricidal agents**
1,4-Diaryl-2-fluoro-2-butene als insectizide und akarizide Mittel
1,4-diaryl-2-fluoro-2-buténes comme agents insecticides et acaricides

(30) Priority: 03.06.1996 US 660221; 17.03.1997 US 819623
(43) Date of publication of application: 10.12.1997
(73) Proprietor: BASF AKTIENGESELLSCHAFT, 67056 Ludwigshafen (DE)
(72) Inventor: Barnes, Keith Douglas, Newtown, Pennsylvania 18940 (US); Hu, Yulin, Plainsboro, New Jersey 08536 (US)
(74) Representative: Köster, Reinhold, Dr.

(56) References cited:
- WO-A-94/06741
- GB-A- 2 288 803
- US-A- 4 975 451

## Description

Insect and acarid pests destroy growing and harvested crops. In the United States, agronomic crops must compete with thousands of those pests. In particular, tobacco budworms and southern armyworms are especially devastating to crops.

Tobacco budworms cause tremendous economic losses in agronomic crops. In particular, budworms devastate cotton crops by feeding on green bolls. Control of budworms is complicated by their resistance to many common insecticides, including organophosphates, carbamates and pyrethroids.

In spite of the commercial insecticides and acaricides available today, damage to crops, both growing and harvested, caused by insect and acarid pests still occurs. Accordingly, there is ongoing research to create new and more effective insecticidal and acaricidal agents.

Certain fluoroolefin compounds are known to possess insecticidal and acaricidal activity (see, e.g., U.S. 5,248,834; GB 2,288,803-A and WO 94/06741). The fluoroolefin compounds disclosed in GB 2,288,803-A and WO 94/06741 are outside the scope of the present invention. U.S. 5,248,834 generically discloses certain 1-aryl-1-(3-aryl-1-fluoroprop-1-enyl)cyclopropane compounds. However, that patent does not provide a method to prepare those compounds. In fact, U.S. 5,248,834 does not provide a method to prepare any fluoroolefin compounds.

It is, therefore, an object of the present invention to provide compounds which are highly effective for the control of insect and acarid pests.

It is also an object of the present invention to provide a method for the control of insect and acarid pests.

It is a further object of this invention to provide a method for the protection of growing and harvested crops from damage caused by insect and acarid attack and infestation.

These and other objects of the present invention will become more apparent from the detailed description thereof set forth below.

### SUMMARY OF THE INVENTION

The present invention comprises 1,4-diaryl-2-fluoro-2-butene compounds which are useful as insecticidal and acaricidal agents. Those compounds are also useful for protecting-plants from damage caused by insect and acarid attack and infestation.

The 1,4-diaryl-2-fluoro-2-butene compounds of the present invention have the structural formula I 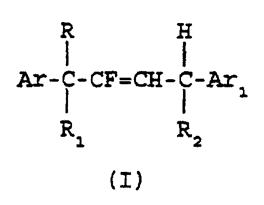 wherein
- Ar: is phenyl optionally substituted with any combination of from one to three halogen, C₁-C₄alkyl, C₁-C₄haloalkyl, C₁-C₄alkoxy or C₁-C₄haloalkoxy groups,
1- or 2-naphthyl optionally substituted with any combination of from one to three halogen, C₁-C₄alkyl, C₁-C₄haloalkyl, C₁-C₄alkoxy or C₁-C₄haloalkoxy groups, or
a 5- or 6-membered heteroaromatic ring optionally substituted with any combination of from one to three halogen, C₁-C₄alkyl, C₁-C₄haloalkyl, C₁-C₄alkoxy or C₁-C₄haloalkoxy groups;
- R and R₁: are each independently hydrogen, C₁-C₄alkyl, C₁-C₄haloalkyl, C₃-C₆cycloalkyl or C₃-C₆halocycloalkyl;
- R₂: is hydrogen, Cl, Br, cyano or OR₃;
- R₃: is hydrogen or C₁-C₄alkyl; and
- Ar₁: is phenoxyphenyl optionally substituted with any combination of from one to six halogen, C₁-C₄-alkyl, C₁-C₄haloalkyl, C₁-C₄alkoxy or C₁-C₄haloalkoxy groups,
phenyl optionally substituted with any combination of from one to five halogen, C₁-C₄alkyl, C₁-C₄haloalkyl, C₁-C₄alkoxy or C₁-C₄haloalkoxy groups,
biphenyl optionally substituted with any combination of from one to five halogen, C₁-C₄alkyl, C₁-C₄haloalkyl, C₁-C₄alkoxy or C₁-C₄haloalkoxy groups,
phenoxypyridyl optionally substituted with any combination of form one to five halogen, C₁-C₄alkyl, C₁-C₄haloalkyl, C₁-C₄alkoxy or C₁-C₄haloalkoxy groups,
benzylpyridyl optionally substituted with any combination of from one to five halogen, C₁-C₄alkyl, C₁-C₄-haloalkyl, C₁-C₄alkoxy or C₁-C₄haloalkoxy groups,
benzylphenyl optionally substituted with any combination of from one to five halogen, C₁-C₄alkyl, C₁-C₄haloalkyl, C₁-C₄alkoxy or C₁-C₄haloalkoxy groups,
benzoylphenyl optionally substituted with any combination of from one to five halogen, C₁-C₄alkyl, C₁-C₄haloalkyl, C₁-C₄alkoxy or C₁-C₄haloalkoxy groups,
1- or 2-naphthyl optionally substituted with any combination of from one to three halogen, C₁-C₄alkyl, C₁-C₄haloalkyl, C₁-C₄alkoxy or C₁-C₄haloalkoxy groups, or
a 5- or 6-membered heteroaromatic ring optionally substituted with any combination of from one to three halogen, C₁-C₄alkyl, C₁-C₄haloalkyl, C₁-C₄alkoxy or C₁-C₄haloalkoxy groups, and
the optical isomers thereof, and
the cis and trans isomers thereof.

This invention also comprises compositions containing those compounds and methods for using those compounds and compositions. Advantageously, it has been found that the 1,4-diaryl-2-fluoro-2-butene compounds of the present invention, and compositions containing them, are useful for the control of insect and acarid pests. The compounds of this invention are also useful for the protection of plants from damage caused by insect and acarid attack and infestation.

### DETAILED DESCRIPTION OF THE INVENTION

The present invention provides a method for the control of insect or acarid pests which comprises contacting said pests or their food supply, habitat or breeding grounds with a pesticidally effective amount of a 1,4-diaryl-2-fluoro-2-butene compound of formula I.

The present invention also provides a method for the protection of growing plants from attack or infestation by insect or acarid pests which comprises applying to the foliage of the plants, or to the soil or water in which they are growing, a pesticidally effective amount of a 1,4-diaryl-2-fluoro-2-butene compound of formula I.

The 1,4-diaryl-2-fluoro-2-butene compounds of the present invention have the structural formula I 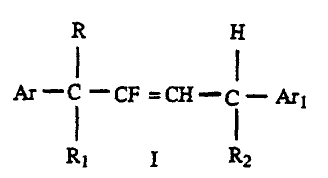 wherein Ar, Ar₁, R, R₁ and R₂ are as described hereinabove for formula I.

In formula I above, the 5- and 6-membered heteroaromatic ring may suitably be a ring containing 1 to 4 heteroatoms selected from N,O and S, wherein the heteroatoms may be the same or different, e.g. the rings include, but are not limited to, pyridyl, pyrazolyl, imidazolyl, triazolyl, isoxazolyl, tetrazolyl, pyrazinyl, pyridazinyl, triazinyl, furanyl, thienyl and thiazolyl rings each optionally substituted as described in formula I above.

Exemplary of halogen hereinabove are fluorine, chlorine, bromine and iodine. The terms "C₁-C₄haloalkyl", "C₃-C₆halocycloalkyl" and "C₁-C₄haloalkoxy" are defined as a C₁-C₄alkyl group, a C₃-C₆cycloalkyl group and a C₁-C₄alkoxy group substituted with one or more halogen atoms, respectively, wherein the halogen atoms may be the same or different.

When used herein as a group or part of a group the term alkyl includes straight or branched chain alkyl groups such as methyl, ethyl, n-propyl, isopropyl, n-butyl, isobutyl, s-butyl and t-butyl. When used herein as a group or part of a group the term cycloalkyl includes cyclopropyl, cyclobutyl and cyclohexyl.

Groups containing two or more rings, such as biphenyl, phenoxypyridyl and benzylphenyl, which may be substituted may be substituted on either ring unless otherwise specified herein.
Preferred formula I 1,4-diaryl-2-fluoro-2-butene compounds of the present invention are those wherein
- Ar: is phenyl optionally substituted with any combination of from one to three halogen, C₁-C₄alkyl, C₁-C₄haloalkyl, C₁-C₄alkoxy or C₁-C₄haloalkoxy groups;
- R and R₁: are each independently hydrogen, C₁-C₄alkyl, C₁-C₄haloalkyl, C₃-C₆cycloalkyl or C₃-C₆halocycloalkyl provided that at least one of R and R₁ is other than hydrogen;
- R₂ is: hydrogen, Cl, Br, cyano or OR₃;
- R₃ is: hydrogen or C₁-C₄alkyl; and
- Ar₁ is: 3-phenoxyphenyl optionally substituted with any combination of from one to six halogen, C₁-C₄alkyl, C₁-C₄haloalkyl, C₁-C₄alkoxy or C₁-C₄haloalkoxy groups,
3-biphenyl optionally substituted with any combination of from one to five halogen, C₁-C₄alkyl, C₁-C₄haloalkyl, C₁-C₄alkoxy or C₁-C₄haloalkoxy groups, or
3-benzylphenyl optionally substituted with any combination of from one to five halogen, C₁-C₄alkyl, C₁-C₄haloalkyl, C₁-C₄alkoxy or C₁-C₄haloalkoxy groups.
More preferred insecticidal and acaricidal agents of the present invention are those wherein
- Ar: is phenyl optionally substituted with any combination of from one to three halogen, C₁-C₄alkyl, C₁-C₄haloalkyl, C₁-C₄alkoxy or C₁-C₄haloalkoxy groups;
- R: is isopropyl or cyclopropyl and R₁ is hydrogen, or R and R₁ are methyl;
- R₂: is hydrogen; and
- Ar₁: is 3-phenoxyphenyl optionally substituted with any combination of from one to six halogen, C₁-C₄alkyl, C₁-C₄haloalkyl, C₁-C₄alkoxy or C₁-C₄haloalkoxy groups.
Most preferred 1,4-diaryl-2-fluoro-2-butene compounds of this invention are those wherein
- Ar: is phenyl optionally substituted with any combination of from one to three halogen, C₁-C₄alkyl, C₁-C₄haloalkyl, C₁-C₄alkoxy or C₁-C₄haloalkoxy groups,
- R: is cyclopropyl and R₁ is hydrogen;
- R₂: is hydrogen; and
- Ar₁: is 3-phenoxyphenyl optionally substituted with any combination of from one to six halogen, C₁-C₄alkyl, C₁-C₄haloalkyl, C₁-C₄alkoxy or C₁-C₄haloalkoxy groups.

Formula I compounds of this invention which are particularly effective insecticidal agents include
1- [1- (*p*-chlorophenyl) -2-fluoro-4- (4-fluoro-3-phenoxyphenyl) -2-butenyl]cyclopropane, (R, S) - (Z) -;
1-[1-(*p*-chlorophenyl) -2-fluoro-4-(*m*-phenoxyphenyl)-2-butenyl]cyclopropane, (R,S)-(Z)-;
4-(*p*-chlorophenyl)-3-fluoro-1-(4-fluoro-3-phenoxyphenyl)-5-methyl-2-hexene, (R,S)-(Z)-;
4-(*p*-chlorophenyl)-3-fluoro-5-methyl-1-(*m*-phenoxyphenyl)-2-hexene, (R,S)-(Z)-;
4-(*p*-ethoxyphenyl)-3-fluoro-1-(4-fluoro-3-phenoxyphenyl)-5-methyl-2-hexene, (R,S)-(Z)-;
1-[1-(*p*-ethoxyphenyl)-2-fluoro-4-(4-fluoro-3-phenoxyphenyl)-2-butenyl]cyclopropane, (R,S)-(Z)-;
4-(*p*-ethoxyphenyl)-3-fluoro-5-methyl-1-(*m*-phenoxyphenyl)-2-hexene, (R,S)-(Z)-;
4-(*p*-ethoxyphenyl)-3-fluoro-1- [*m*-(*p*-fluorophenoxy)-phenyl]-5-methyl-2-hexene, (R,S)-(Z)-;
1-{1-(*p*-chlorophenyl)-2-fluoro-4- [*m*-(*p*-fluorophenoxy)-phenyl]-2-butenyl}cyclopropane, (R,S)-(Z)-;
1-[2-fluoro-4-(4-fluoro-3-phenoxyphenyl)-1-(*p*-fluorophenyl)-2-butenyl]cyclopropane, (R,S)-(Z)-;
1-[1-(*p*-ethoxyphenyl)-2-fluoro-4-(*m*-phenoxyphenyl)-2-butenyl]cyclopropane, (R,S)-(Z)-;
1-[2-fluoro-1-(*p*-fluorophenyl)-4-(*m*-phenoxyphenyl)-2-butenyl]cyclopropane, (R,S)-(Z)-;
4-(*p*-chlorophenyl)-3-fluoro-4-methyl-1-(*m*-phenoxyphenyl)-2-pentene, (Z)-;
4-(*p*-chlorophenyl)-3-fluoro-1-(4-fluoro-3-phenoxyphenyl)-4-methyl-2-pentene, (Z)-;
1-(*p*-chlorophenyl)-1-[1-fluoro-3-(*m*-phenoxyphenyl) propenyl]cyclopropane, (Z)-; and
1-(*p*-chlorophenyl)-1-[1-fluoro-3- (4-fluoro-3-phenoxyphenyl)propenyl]cyclopropane, (Z)-, among others.

Flow Diagram I illustrates a method for preparing 1,4-diaryl-2-fluoro-2-butene compounds of the present invention wherein R and R₁ are each independently C₁-C₄alkyl, C₁-C₄haloalkyl, C₃-C₆cycloalkyl or C₃-C₆halocycloalkyl; R₂ is hydrogen; and the double bond is in the (Z)-configuration. This method comprises: reacting an arylacetonitrile of formula II with a selective reducing agent, such as diisobutylaluminium hydride, and quenching with water to form a 2-arylacetaldehyde of formula III; reacting the formula III compound with a zinc/triphenyl phosphine/carbon tetrabromide mixture to form a 3-aryl-1,1,-dibromo-1-propene of formula IV; reacting the formula IV compound with a base such as an alkyllithium and phenyl cyanate to form a 4-aryl-2-butenenitrile of formula V; reacting the formula V compound with cesium fluoride, potassium hydrogen fluoride and water in N,N-dimethylformamide to form a 4-aryl-3-fluoro-2-butenenitrile, (Z)- of formula VI; selectively reducing the formula VI compound with a reducing agent such as diisobutylaluminium hydride and quenching with water to form a 4-aryl-3-fluoro-2-butenal, (Z)- of formula VII; reducing the formula VII compound with a conventional reducing agent such as lithium aluminum hydride or sodium borohydride to form a 4-aryl-3-fluoro-2-buten-1-ol, (Z)- of formula VIII; reacting the formula VIII compound with a brominating agent, such as a triphenyl phosphine and bromine mixture, in the presence of a solvent, such as a halogenated hydrocarbon, to form a 4-aryl-1-bromo-3-fluoro-2-butene, (Z)- of formula IX; and reacting the formula IX compound with about 0.0025 to 0.1 molar equivalent of a palladium catalyst, such as bis(dibenzylideneacetone)palladium (0) (Pd(dba)₂), bis(acetonitrile)palladium(II)chloride, bis(triphenylphosphine)palladium(II)chloride, tetrakis(triphenylphosphine)palladium(0) and the like, at least about 2 molar equivalents of a base, such as an alkali metal carbonate, an alkaline earth metal carbonate, an alkali metal hydrogen carbonate, an alkali metal hydroxide, an alkaline earth metal hydroxide, an alkali metal C₁-C₆alkoxide and the like, and a boronic acid of formula X in the presence of a solvent, such as an aromatic hydrocarbon, a halogenated aromatic hydrocarbon, a C₁-C₄alcohol, and the like, and mixtures thereof.

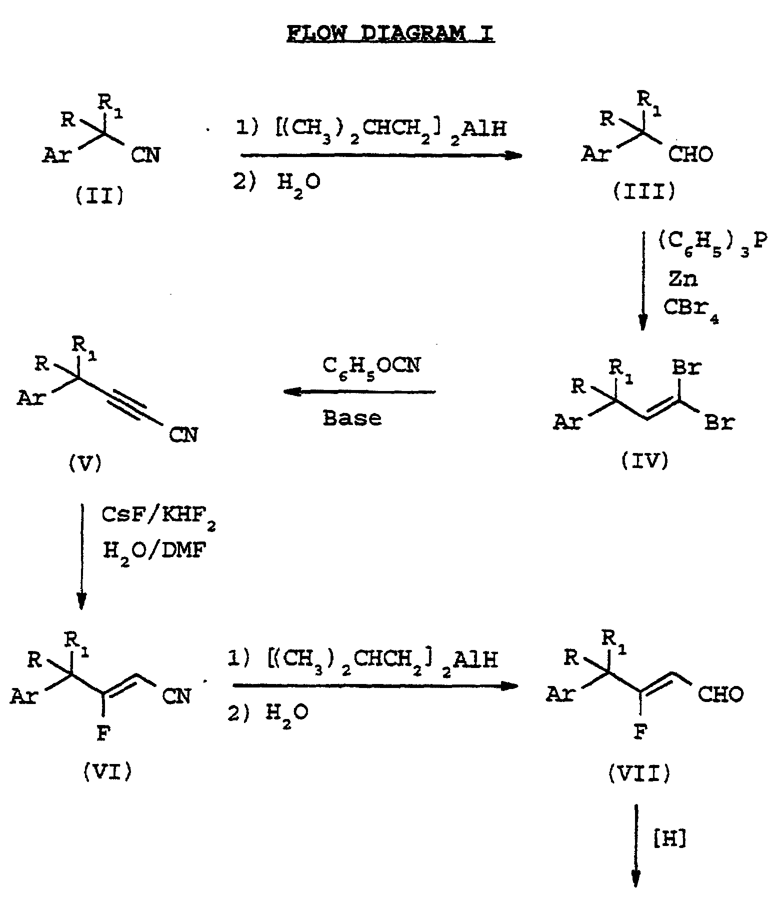

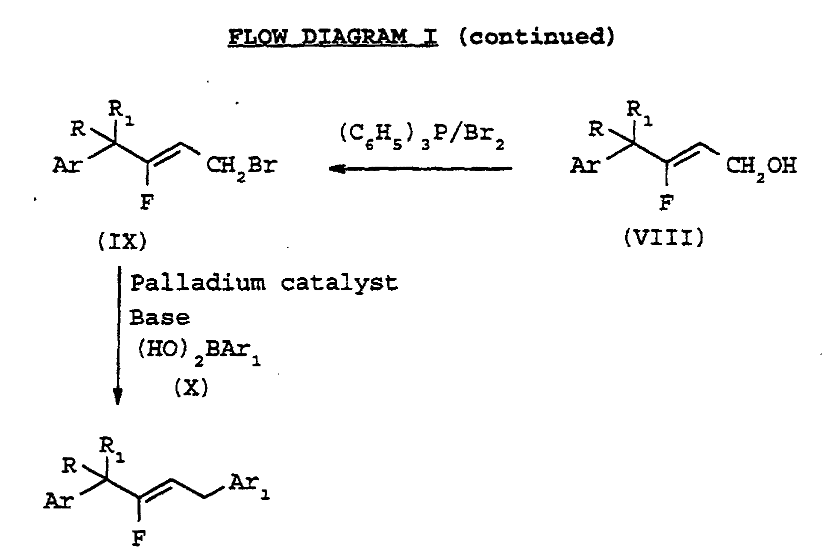

Formula I compounds wherein the double bond is in the (E)- configuration may be prepared by isomerizing certain intermediate compounds described hereinabove which are predominantly in the (Z)- configuration using conventional procedures such as exposure to light.

Formula I compounds wherein R₁ and R₂ are hydrogen may be prepared, as indicated in Flow Diagram II, by reacting a ketone or aldehyde of formula XI with an ester of formula XII in the presence of a base such as sodium hydride or lithium diisopropylamide to form a 3-aryl-2-fluoro-2-propenate of formula XIII, reducing the formula XIII compound with a reducing agent such as lithium aluminum hydride to form a 3-aryl-2-fluoro-2-propen-1-ol of formula XIV, oxidizing the formula XIV compound with an oxidizing agent such as manganese(IV) oxide to form a 3-aryl-2-fluoro-2-propenal of formula XV, reacting the formula XV compound with an ylide of formula XVI in the presence of a base such as sodium hydride, an alkyllithium or lithium diisopropylamide to form a diene of formula XVII, and reacting the formula XVII compound with magnesium in the presence of a protic solvent such as a C₁-C₄alcohol.

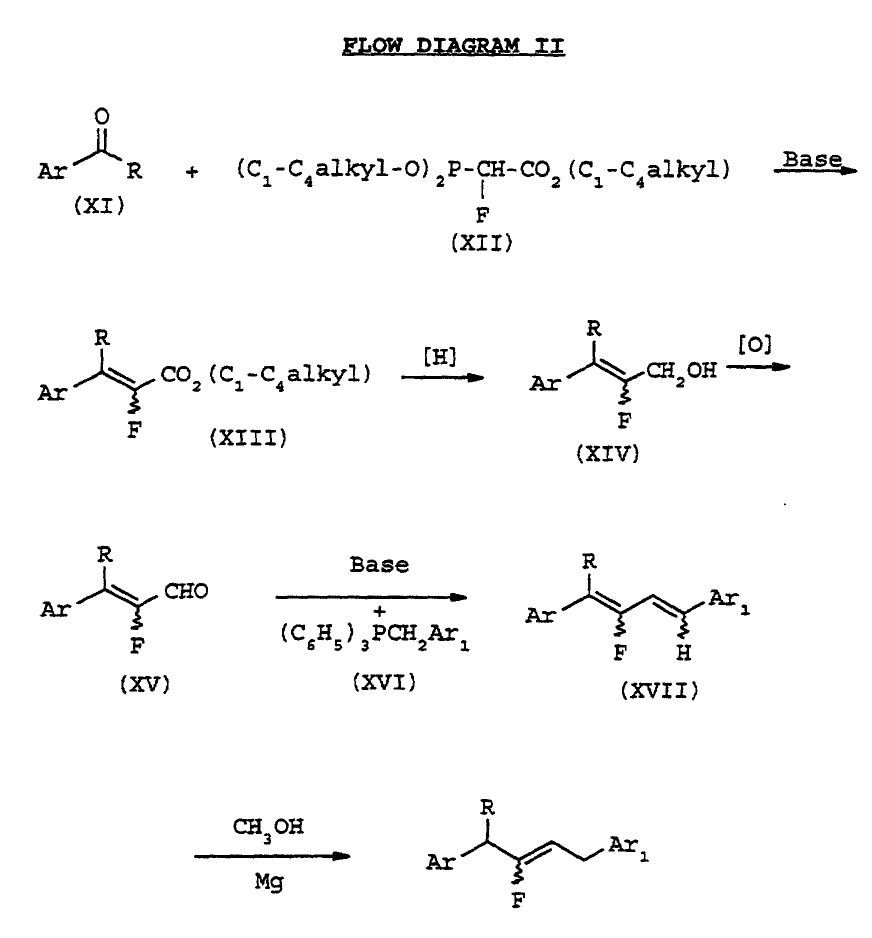

Alternatively, formula XVII diene compounds may be prepared, as indicated in Flow Diagram III, by reacting a 3-aryl-2-fluoro-2-propen-1-ol of formula XIV with thionyl chloride in the presence of a solvent such as pyridine to form a 3-aryl-1-chloro-2-fluoro-2-propene of formula XVIII, reacting the formula XVIII compound with triphenylphosphine to form an ylide of formula XIX, and reacting the formula XIX compound with a base such as sodium hydride and an aldehyde of formula XX.

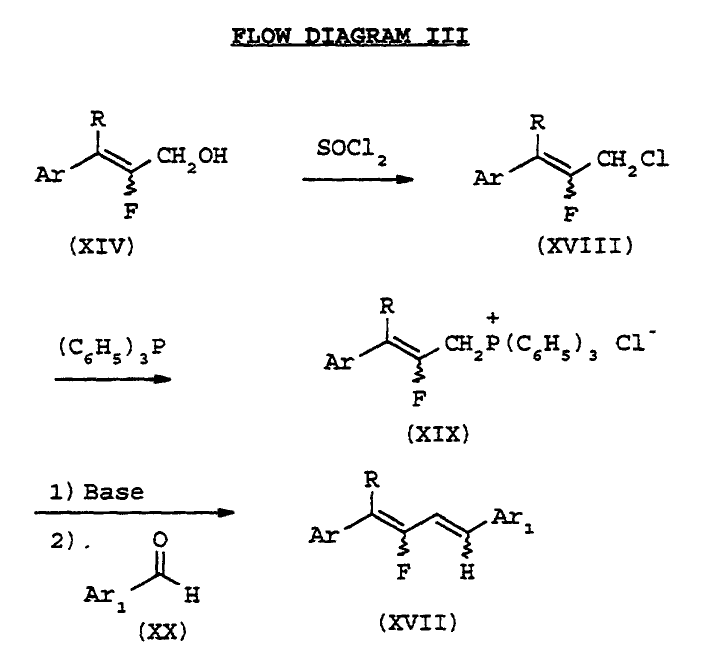

Advantageously, intermediate 3-aryl-2-fluoro-2-propenal compounds of formula XV may be prepared, as illustrated in Flow Diagram IV, by reacting an aldehyde or ketone of formula XI with an alkoxymethyl triphenyl phosphonium halide of formula XXI in the presence of a base such as butyllithium to form a 2-arylvinyl methyl ether of formula XXII, reacting the formula XXII compound with dichlorofluoromethane and a base such as potassium hydroxide in the presence of water and optionally a phase transfer catalyst such as 18-crown-6 to form an intermediate, and reacting the intermediate *in situ* with water at an elevated temperature, preferably about 60 to 90 °C.

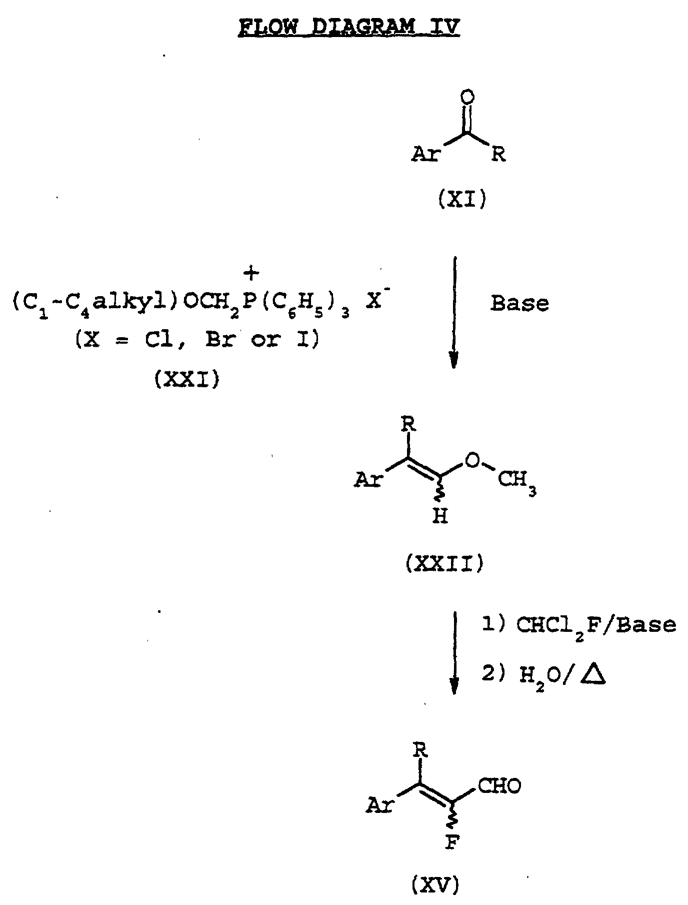

Formula I compounds wherein R₂ is Cl or Br may be prepared by halogenating a formula I compound wherein R₂ is hydrogen with a chlorinating agent such as N-chlorosuccinimide or a brominating agent such as N-bromosuccinimide. The reaction scheme is shown below in Flow Diagram V.

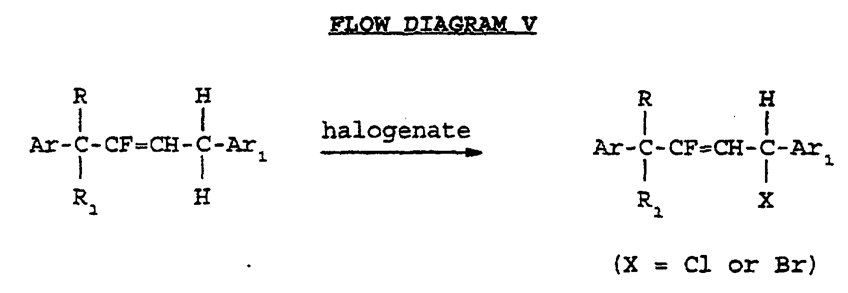

Advantageously, formula I compounds wherein R₂ is cyano may be prepared by reacting a formula I compound wherein R₂ is Cl or Br with sodium cyanide. The reaction is shown in Flow Diagram VI.

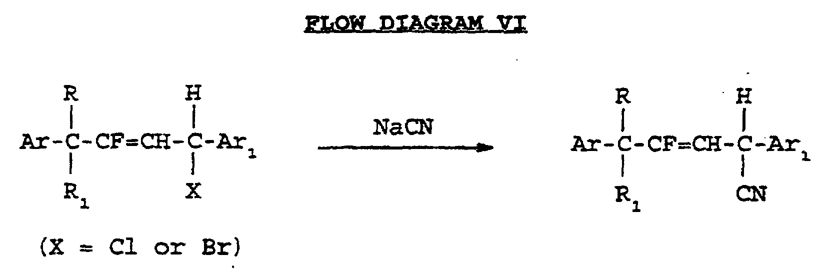

1,4-Diaryl-2-fluoro-2-butene compounds of formula I wherein R₂ is OR₃ may be prepared as shown below in Flow Diagram VII.

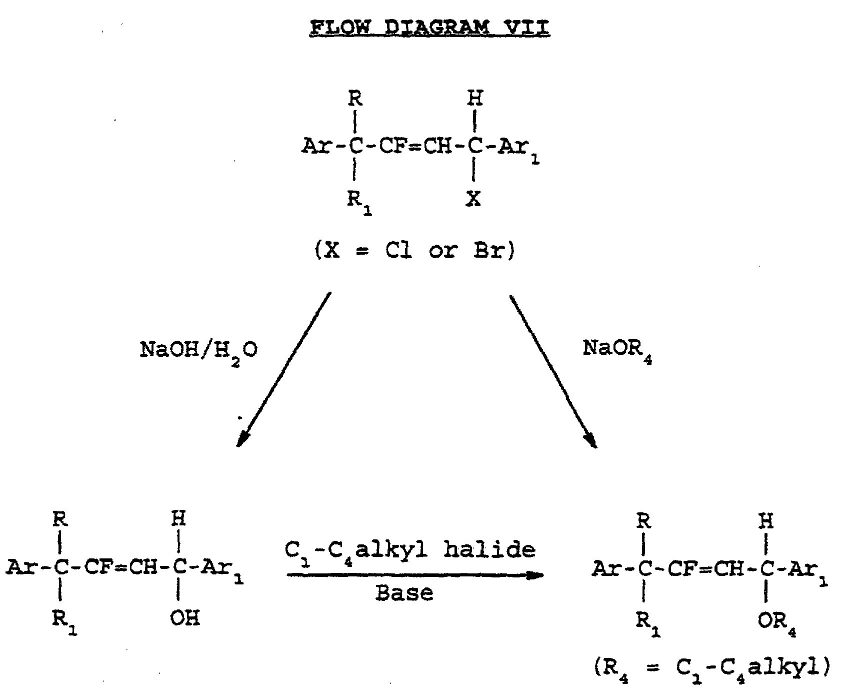

Formula I compounds wherein R₂ is Cl, Br or OR₃, and R and R₁ are other than hydrogen may be prepared as shown in Flow Diagram VIII.

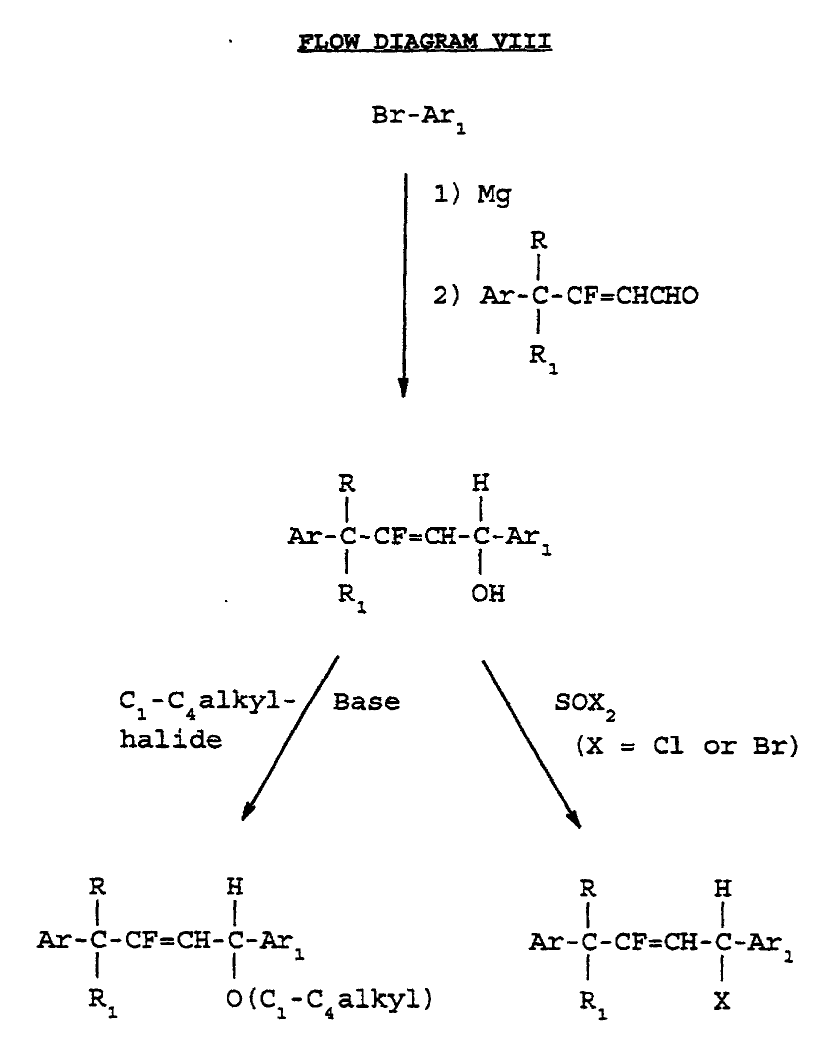

The 1,4-diaryl-2-fluoro-2-butene compounds of the present invention are effective for controlling insect and acarid pests. Those compounds are also effective for protecting growing or harvested crops from damage caused by insect and acarid attack and infestation.

Insects controlled by the 1,4-diaryl-2-fluoro-2-butene compounds of this invention include Lepidoptera such as tobacco budworms, cabbage loopers, cotton boll worms, beet armyworms, southern armyworms and diamondback moths; Homoptera such as aphids, leaf hoppers, plant hoppers and white flies; Thysanoptera such as thrips; Coleoptera such as boll weevils, Colorado potato beetles, southern corn rootworms, western corn rootworms and mustard beetles; and Orthoptera such as locusts, crickets, grasshoppers and cockroaches. Acarina controlled by the compounds of this invention include mites such as two-spotted spider mites, carmine spider mites, banks grass mites, strawberry mites, citrus rust mites and leprosis mites.

In practice generally about 10 ppm to about 10,000 ppm and preferably about 100 ppm to about 5,000 ppm of a formula I compound, dispersed in water or another liquid carrier, is effective when applied to plants or the soil in which the plants are growing to protect the plants from insect and acarid attack and infestation.

The 1,4-diaryl-2-fluoro-2-butene compounds of this invention are also effective for controlling insect and acarid pests when applied to the foliage of plants and/or to the soil or water in which said plants are growing in sufficient amount to provide a rate of about 0.1 kg/ha to 4.0 kg/ha of active ingredient.

While the compounds of this invention are effective for controlling insect and acarid pests when employed alone, they may also be used in combination with other biological chemicals, including other insecticides and acaricides. For example, the formula I compounds of this invention may be used effectively in conjunction or combination with pyrethroids, phosphates, carbamates, cyclodienes, endotoxin of *Bacillus thuringiensis* (Bt), formamidines, phenol tin compounds, chlorinated hydrocarbons, benzoylphenyl ureas, pyrroles and the like.

The compounds of this invention may be formulated as emulsifiable concentrates, flowable concentrates or wettable powders-which are diluted with water or other suitable polar solvent, generally *in situ*, and then applied as a dilute spray. Said compounds may also be formulated in dry compacted granules, granular formulations, dusts, dust concentrates, suspension concentrates, microemulsions and the like all of which lend themselves to seed, soil, water and/or foliage applications to provide the requisite plant protection. Such formulations or compositions of the present invention include a compound of the invention (or combinations thereof) admixed with one or more agronomically acceptable inert, solid or liquid carriers. Those compositions contain a pesticidally effective amount of said compound or compounds, which amount may vary depending upon the particular compound, target pest, and method of use. Those skilled in the art can readily determine what is a pesticidally effective amount without undue experimentation.

In order to facilitate a further understanding of the invention, the following examples are presented primarily for the purpose of illustrating more specific details thereof. The scope of the invention should not be deemed limited by the examples, but encompasses the entire subject matter defined in the claims.

### EXAMPLE 1

### Preparation of p-Chloro-α-methylhydratroponitrile

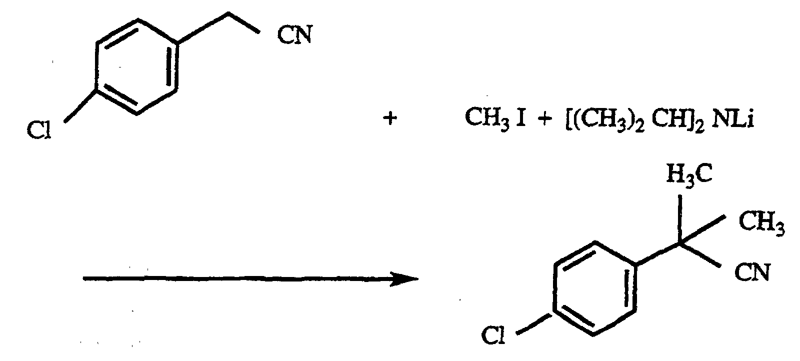

A solution of (*p*-chlorophenyl)acetonitrile (30.32 g, 0.20 mol) in tetrahydrofuran is treated dropwise with lithium diisopropylamide (0.44 mol, 220 mL of a 2 M solution in heptane/tetrahydrofuran/benzene) at -25 °C to -30 °C over 60 minutes under nitrogen, stirred at -15 °C for one hour, treated dropwise with a solution of iodomethane (62.45 g, 0.44 mol) in tetrahydrofuran at -15 °C, stirred at -15 °C for one hour, and diluted with water. The aqueous solution is extracted with ether. The organic extract is washed sequentially with water, 2 N hydrochloric acid and water, dried over anhydrous sodium sulfate, and concentrated *in vacuo* to obtain an oily residue. The residue is distilled to give the title product as a colorless oil (32.6 g, bp 89-91 °C/1 mm Hg, 90.7% yield).

### EXAMPLE 2

### Preparation of 2-(p-Chlorophenyl)-2-methylpropionaldehyde

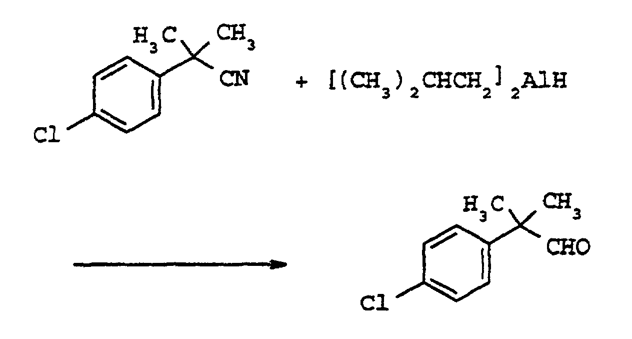

Diisobutylaluminum hydride (0.236 mol, 236 mL of a 1 M solution in hexane) is added over 90 minutes to a solution of *p*-chloro-α-methylhydratroponitrile (32.6 g, 0.181 mol) in diethyl ether under nitrogen at 0 °C. After the addition is complete, water and 6 N hydrochloric acid are added to the reaction mixture while maintaining the temperature below 30 °C. The resultant aqueous solution is stirred overnight at room temperature and extracted with diethyl ether. The organic extracts are combined, washed sequentially with 2 N hydrochloric acid and water, dried over anhydrous sodium sulfate, and concentrated *in vacuo* to give the title product as an oil (31.1 g, 94% yield).

Using essentially the same procedure, but substituting 1-(*p*-chlorophenyl)cyclopropanecarbonitrile for *p*-chloro-α-methylhydratroponitrile, 1-(*p*-chlorophenyl)cyclopropanecarboxaldehyde is obtained as a colorless solid, mp 38-41 °C.

### EXAMPLE 3

### Preparation of 1,1-Dibromo-3-(p-chlorophenyl)-3-methyl-1-butene

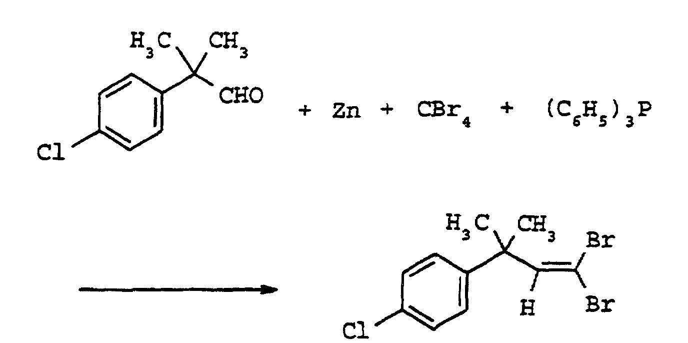

A solution of triphenyl phosphine (89.34 g, 0.34 mol) in methylene chloride is added dropwise to a mixture of zinc powder (22.27 g, 0.34 mol) and carbon tetrabromide (112.8 g, 0.34 mol) in methylene chloride at 20 °C over one hour. The resultant mixture is stirred at room temperature overnight, treated dropwise with a solution of 2-(*p*-chlorophenyl)-2-methylpropionaldehyde (31.1 g, 0.17 mol) in methylene chloride over 20 minutes, refluxed for 2 days, and poured into petroleum ether. The organic mixture is filtered through diatomaceous earth and concentrated *in vacuo* to obtain a residue. The residue is distilled to give the title product as an oil (38.5 g, bp 121-123 °C/0.3 mm Hg, 66.8% yield).

Using essentially the same procedure, but substituting 1-(*p*-chlorophenyl)cyclopropanecarboxaldehyde for 2-(*p*-chlorophenyl-2-methylpropionaldehyde, 1-(2,2-dibromovinyl) -1-(*p*-chlorophenyl) cyclopropane is obtained as a colorless oil.

### EXAMPLE 4

### Preparation of 4-(p-Chlorophenyl)-4-methyl-2-pentynenitrile

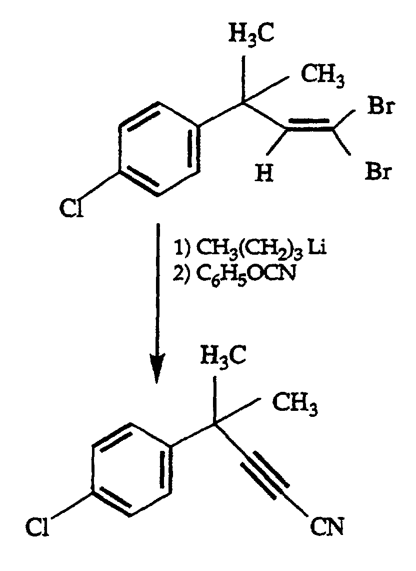

A solution of 1,1-dibromo-3-(*p*-chlorophenyl)-3-methyl-1-butene (38.5 g, 0.113 mol) in tetrahydrofuran is treated with n-butyllithium (0.25 mol, 100 mL of a 2.5 M solution in hexane) under nitrogen over 45 minutes while maintaining the temperature below -65 °C, stirred overnight at dry ice/acetone bath temperature, treated dropwise with a solution of phenyl cyanate (14.89 g, 0.125 mol) in tetrahydrofuran over 30 minutes at -65 °C to -70 °C, allowed to warm to 10 °C, and diluted with ethyl acetate and 5% sodium hydroxide solution. The resultant mixture is extracted with ethyl acetate. The organic extracts are combined, washed sequentially with 5% sodium hydroxide solution and water, dried over anhydrous sodium sulfate and concentrated *in vacuo* to obtain a residue. The residue is distilled to give the title product as an oil (18.7 g, bp 110-113 °C/0.9 mm Hg, 80.7% yield). Using essentially the same procedure, but substituting 1-(2,2-dibromovinyl)-1-(*p*-chlorophenyl) cyclopropane for 1,1-dibromo-3-(*p*-chlorophenyl)-3-methyl-1-butene, 3-[1-(*p*-chlorophenyl)cyclopropyl]-2-propyne-1-carbonitrile is obtained as a yellow solid, mp 62-64 °C.

### EXAMPLE 5

### Preparation of 4-(p-Chlorophenyl)-3-fluoro-4-methyl-2-pentenenitrile, (Z)-

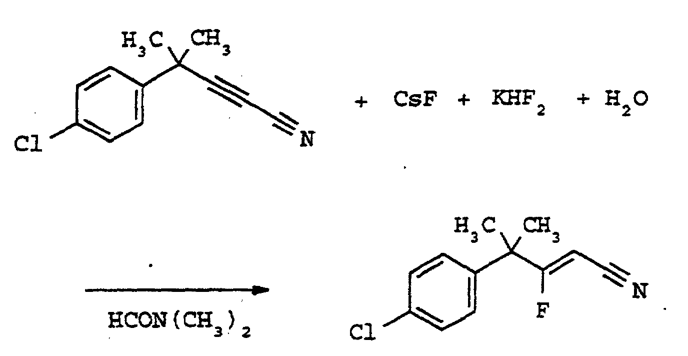

A mixture of cesium fluoride (41.38 g, 0.272 mol), potassium hydrogen fluoride (10.64 g, 0.136 mol) and water (13.07 g, 0.726 mol) in N,N-dimethylformamide is stirred for 10 minutes, treated with a solution of 4-(*p*-chlorophenyl) -4-methyl-2-pentynenitrile (18.5 g, 0.091 mol) in N,N-dimethylformamide, stirred at 80-85 °C for 4 hours, stirred at room temperature overnight and diluted with water. The aqueous mixture is extracted with ethyl acetate. The organic extracts are combined, washed with water, dried over anhydrous sodium sulfate and concentrated *in vacuo* to obtain a residue. Column chromatography of the residue using silica gel and a 1:9 ethyl acetate/hexane solution gives the title product as an oil (15.6 g, 76.8% yield).

Using essentially the same procedure, but substituting 3-[1-(*p*-chlorophenyl)cyclopropyl]-2-propyne-1-carbonitrile for 4- (*p*-chlorophenyl)-4-methyl-2-pentynenitrile, 1-(*p*-chlorophenyl)-β-fluorocyclopropaneacrylonitrile having a (Z)- to (E)- ratio of 9:1 is obtained as a colorless oil.

### EXAMPLE 6

### Preparation of 4-(p-Chlorophenyl)-4-methyl-2-pentenal, (Z)-

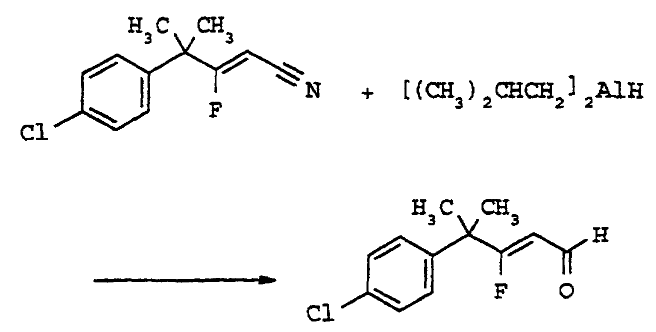

A solution of 4-(*p*-chlorophenyl)-3-fluoro-4-methyl-2-pentenenitrile, (Z)- (15.6 g, 69.7 mmol) in diethyl ether is treated dropwise with diisobutylaluminum hydride (83.6 mmol, 83.6 mL of a 1 M solution in hexane) over 90 minutes at -45 °C under nitrogen, stirred at -40 °C for 35 minutes, and diluted sequentially with water and 2 N hydrochloric acid at -10 °C. The resultant aqueous mixture is stirred at room temperature for one hour and extracted with diethyl ether. The organic extracts are combined, washed sequentially with water, 2 N hydrochloric acid and water, dried over anhydrous sodium sulfate, and concentrated *in vacuo* to give the title product as a brown oil (15.34 g, 97% yield).

Using essentially the same procedure, but substituting 1-(*p*-chlorophenyl)-β-fluorocyclopropaneacrylonitrile having a (Z)- to (E)- ratio of 9:1 for 4- (*p*-chlorophenyl) -3-fluoro-4-methyl-2-pentenenitrile, (Z) -, 1- (*p*-chlorophenyl) -β-fluorocyclopropaneacrylaldehyde, (Z)- is obtained as a colorless oil.

### EXAMPLE 7

### Preparation of 4-(p-Chlorophenyl)-3-fluoro-4-methyl-2-penten-1-ol, (Z)-

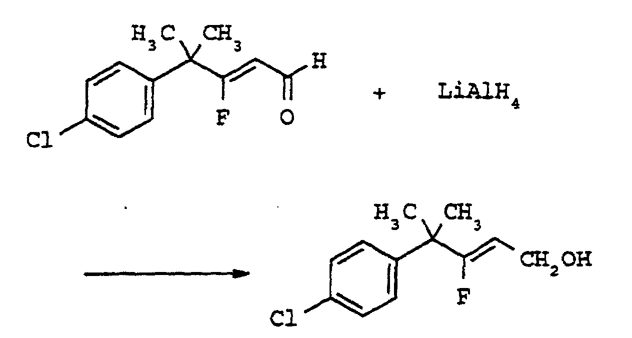

A solution of 4-(*p*-chlorophenyl)-4-methyl-2-pentenal, (Z)- (15.3 g, 67.5 mmol) in diethyl ether is added dropwise over 35 minutes to a mixture of lithium aluminum hydride (1.54 g, 40.5 mmol) in diethyl ether under nitrogen at -60 °C. After the addition is complete, the reaction mixture is stirred for 20 minutes, and diluted sequentially with ethyl acetate, methanol and 2 N hydrochloric acid. The resultant mixture is stirred for 20 minutes and extracted with ethyl acetate. The combined organic extracts are washed sequentially with water and 2 N hydrochloric acid, dried over anhydrous sodium sulfate, and concentrated *in vacuo* to obtain a residue. Flash chromatography of the residue using silica gel and a 2:8 ethyl acetate/hexane solution gives the title product as an oil (10.6 g, 68.7% yield).

Using essentially the same procedure, but substituting 1-(*p*-chlorophenyl)-β-fluorocyclopropaneacrylaldehyde, (Z)- for 4-(*p*-chlorophenyl)-4-methyl-2-pentenal, (Z)-, 1-(*p*-chlorophenyl)-β-fluorocyclopropaneallyl alcohol, (Z)- is obtained as a colorless oil.

### EXAMPLE 8

### Preparation of 1-Bromo-4-(p-chlorophenyl)-3-fluoro-4-methyl-2-pentene, (Z)-

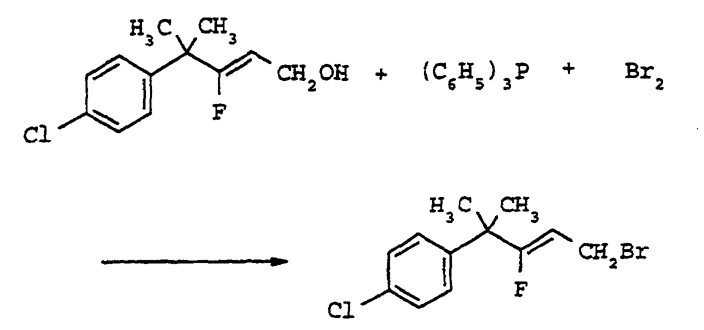

A solution of triphenyl phosphine (12.58 g, 55.6 mmol) in carbon tetrachloride at -5 °C to 5 °C is treated dropwise with a solution of bromine (8.89 g, 55.6 mmol) in carbon tetrachloride over 50 minutes under nitrogen, stirred at room temperature for one hour, treated with a solution of 4-(*p*-chlorophenyl)-3-fluoro-4-methyl-2-penten-1-ol, (Z)- (10.6 g, 46.3 mmol) in carbon tetrachloride over 15 minutes, refluxed for 2.5 hours, cooled to room temperature, and poured into petroleum ether. The resultant mixture is filtered through diatomaceous earth and concentrated *in vacuo* to obtain a residue. Flash chromatography of the residue using silica gel and a 1:9 ethyl acetate/hexane solution gives the title product as an oil (12.5 g, 92.6% yield).

Using essentially the same procedure, but substituting 1-(*p*-chlorophenyl)-β-fluorocyclopropaneallyl alcohol, (Z) - for 4- (*p*-chlorophenyl)-3-fluoro-4-methyl-2-penten-1-ol, (Z)-, 1- (*p*-chlorophenyl)-1-(3-bromo-1-fluoropropenyl)cyclopropane, (Z)- is obtained as a brown oil.

### EXAMPLE 9

### Preparation of 4-Fluoro-3-phenoxybenzeneboronic acid

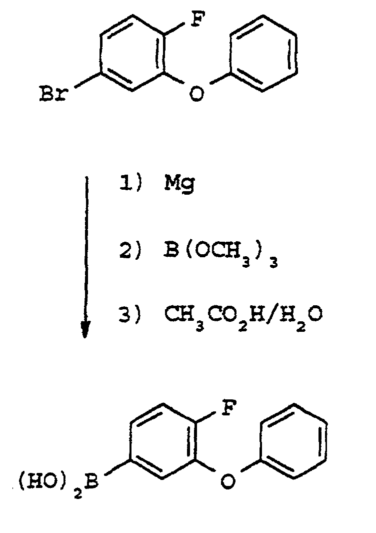

A solution of 5-bromo-2-fluorophenyl phenyl ether (8.01 g, 30 mmol) in tetrahydrofuran is added dropwise over 30 minutes to a mixture of magnesium turnings (0.0802 g, 33 mmol), a crystal of iodine and a few drops of 1,2-dibromoethane in tetrahydrofuran at 50-55 °C under nitrogen. After the addition is complete, the reaction mixture is stirred at 50-55 °C for 70 minutes and cooled to room temperature. The cooled mixture is added over 25 minutes to a solution of trimethyl borate (4.09 mL, 36 mmol) in diethyl ether at dry ice/acetone bath temperature. After the addition is complete, the mixture is stirred at dry ice/acetone bath temperature for 20 minutes, allowed to warm to -10 °C over 25 minutes, diluted sequentially with acetic acid and water, stirred at room temperature for 30 minutes, and extracted with ether. The organic extract is washed with water, dried over anhydrous sodium sulfate and concentrated *in vacuo* to obtain a residue. A mixture of the residue in water is heated over a steam bath for 30 minutes, cooled to room temperature and filtered to obtain a solid which is washed with hexanes and dried to give the title product as a colorless solid (5.7 g, mp 177-180 °C, 82% yield).

Using essentially the same procedure, the following compounds are obtained:

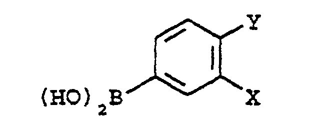

| **X** | **Y** |
|---|---|
| OC₆H₅ | H |
| H | OC₆H₅ |
| Cl | F |
| CH₃ | F |
| H | F |

### EXAMPLE 10

### Preparation of 4-4-(p-Chlorophenyl)-3-fluoro-1-(4-fluoro-3-phenoxyphenyl)-4-methyl-2-pentene, (Z)-

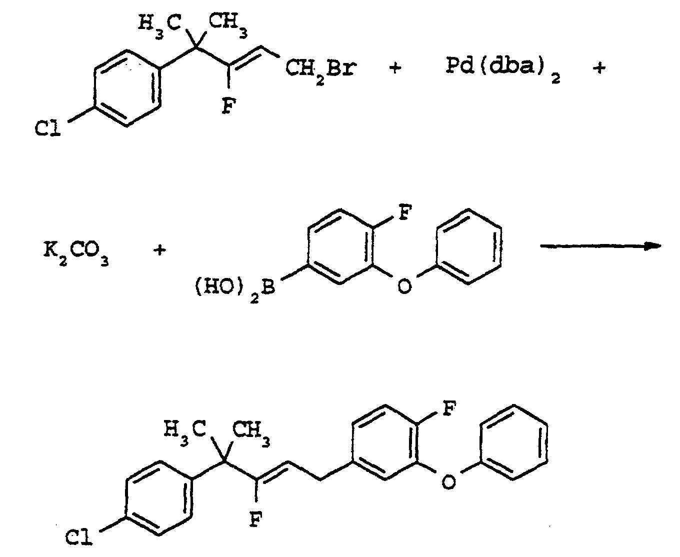

A mixture of 1-bromo-4-(*p*-chlorophenyl)-3-fluoro-4-methyl-2-pentene, (Z)- (1.02 g, 3.5 mmol), and bis(dibenzylideneacetone)palladium(0) (Pd(dba)₂, 0.1 g, 0.17 mmol) in toluene (20 mL) under nitrogen is stirred for one minute, treated with potassium carbonate (1.94 g, 0.014 mol), degassed, treated with a solution of 4-fluoro-3-phenoxybenzeneboronic acid (1.05 g, 4.55 mmol) in ethanol (5 mL), refluxed for 50 minutes, cooled to room temperature, diluted with ethyl acetate and filtered through diatomaceous earth. The filtrate is washed sequentially with water and brine, dried over anhydrous sodium sulfate and concentrated *in vacuo* to obtain a residue. Flash chromatography of the residue using silica gel and a 15:100 methylene chloride/hexane solution gives the title compound as a colorless oil (1.19 g, 85.6% yield) which is identified by NMR spectral analyses. Using essentially the same procedure, the following compounds are obtained:

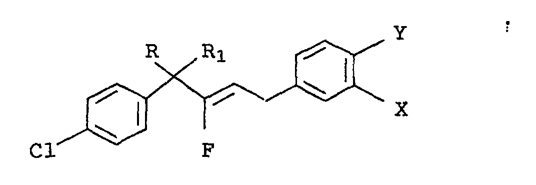

| R | R₁ | X | Y |
|---|---|---|---|
| CH₃ | CH₃ | OC₆H₅ | H¹ |
| CH₃ | CH₃ | H | OC₆H₅ |
| CH₃ | CH₃ | Cl | F |
| CH₃ | CH₃ | CH₃ | F |
| CH₃ | CH₃ | H | F |

| | | | |
|---|---|---|---|
| ¹ Z/E ratio 95:5 | | | |

### EXAMPLE 11

### Preparation of Ethyl p-chloro-β-cyclopropyl-α-fluorocinnamate, (E)- and (Z)-

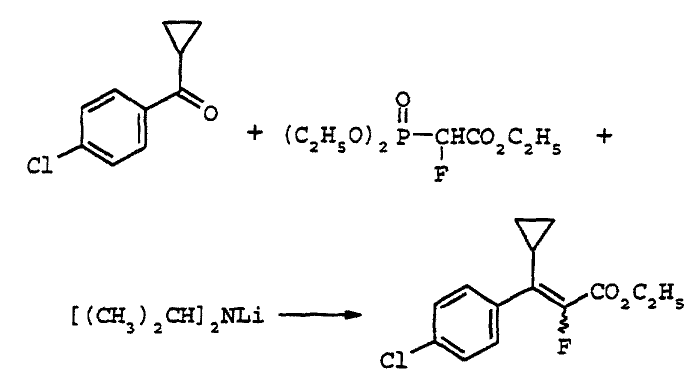

A solution of triethyl 2-fluoro-2-phosphonacetate (49 g, 0.202 mol) in ether is cooled to -55 to -60 °C, treated dropwise over 17 minutes with a 2.0 M solution of lithium diisopropylamide in heptane/tetrahydrofuran/ethylbenzene (116 mL, 0.232 mol), warmed to room temperature over 90 minutes, recooled to -55 to -60 °C, treated over 10 minutes with a solution of 4-chlorophenyl cyclopropyl ketone (36.48 g, 0.202 mol) in ether, stirred at -55 to -60 °C for 20 minutes, warmed to and stirred at room temperature overnight, and quenched with water and 2 N hydrochloric acid (300 mL). The resultant aqueous mixture is extracted with ether. The organic extracts are combined, washed sequentially with water, 2 N hydrochloric acid and water, dried over anhydrous sodium sulfate, and concentrated *in vacuo* to obtain a residue. Kugelrohr distillation of the residue gives the title product as an oil (50 g, 92%, b.p. 100-110 °C/0.5 mmHg).

Using essentially the same procedure, the following compounds are obtained:

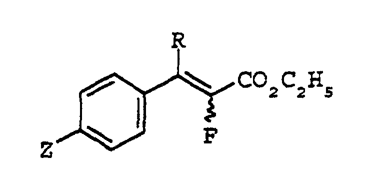

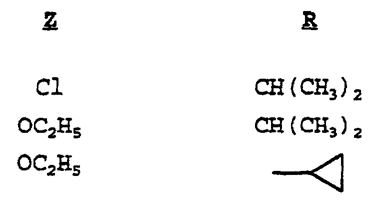

### EXAMPLE 12

### Preparation of 3-(p-Chlorophenyl)-3-cyclopropyl-2-fluoro-2-propen-1-ol, (E)- and (Z)-

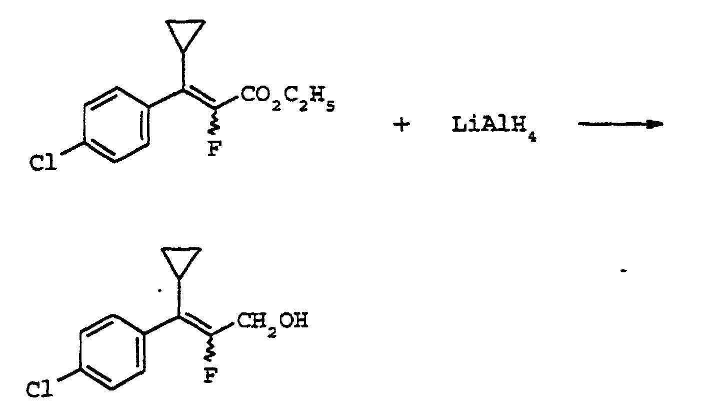

A solution of ethyl *p*-chloro-β-cyclopropyl-α-fluorocinnamate, (E)- and (Z)- (32.25 g, 0.12 mol) in ether is added dropwise to a mixture of lithium aluminum hydride (5.46 g, 0.144 mol) in ether while maintaining the temperature at -55 °C. After the addition is complete, the reaction mixture is warmed to and stirred at -20 °C for 90 minutes, quenched sequentially with ethyl acetate, methanol and 2 N hydrochloric acid, and extracted with ether. The organic extracts are combined, washed sequentially with water, saturated sodium hydrogen carbonate solution and water, dried over anhydrous sodium sulfate, and concentrated *in vacuo* to give the title product as a colorless oil (26.4 g, 97%).

Using essentially the same procedure, the following compounds are obtained:

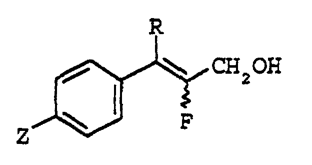

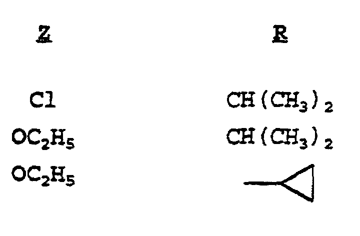

### EXAMPLE 13

### Preparation of p-Chloro-β-cyclopropyl-α-fluorocinnamaldehyde

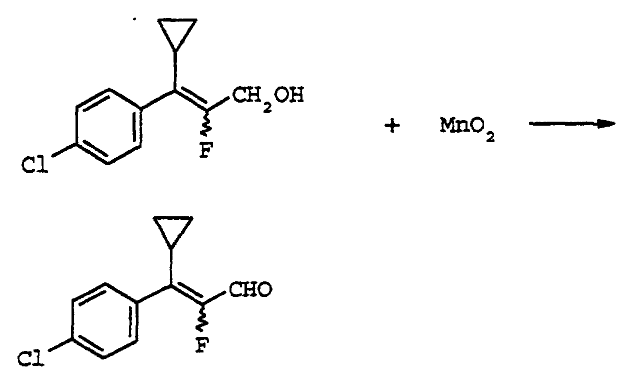

Activated manganese(IV) oxide (101.25 g, 1.16 mol) is added to a solution of 3-(*p*-chlorophenyl)-3-cyclopropyl-2-fluoro-2-propen-1-ol, (E)- and (Z)- (26.4 g, 0.116 mol) in hexanes. The resultant reaction mixture is stirred at room temperature overnight, filtered through a pad of diatomaceous earth, and concentrated *in vacuo* to obtain a residue. Flash column chromatography of the residue using silica gel and an ethyl acetate/hexanes solution (1:9) gives the title product as an oil (15.8 g, 60%).

Using essentially the same procedure, the following compounds are obtained:

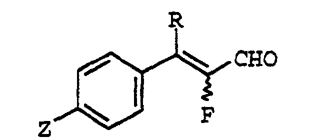

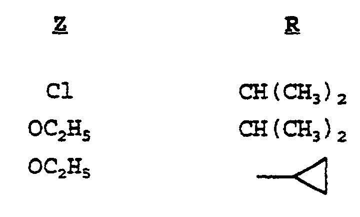

### EXAMPLE 14

### Preparation of 1-[1-(p-Chlorophenyl)-2-methoxyvinyl]-cyclopropane, (E) - and (Z) -

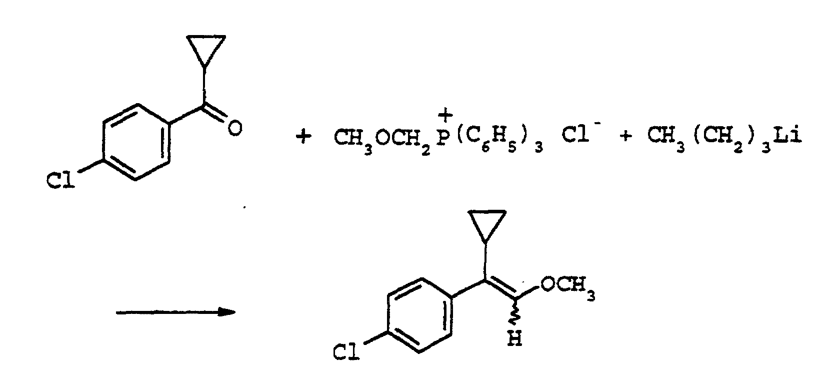

A solution of methoxymethyl triphenyl phosphonium chloride (20.5 g, 0.060 mol) in ether is cooled to -60 °C, treated with a 2.5 M solution of butyllithium in hexanes (25.2 mL, 0.063 mol), warmed to and stirred at 0-5 °C for 90 minutes, recooled to -60 °C, treated with a solution of 4-chlorophenyl cyclopropyl ketone (9.03 g, 0.050 mol) in ether, warmed to and stirred at room temperature overnight, quenched with ethyl acetate and 2 N hydrochloric acid, and extracted with ethyl acetate. The organic extracts are combined, washed sequentially with water, 2 N hydrochloric acid and water, dried over anhydrous sodium sulfate, and concentrated *in vacuo* to obtain a residue. Flash column chromatography of the residue using silica gel and an ethyl acetate/hexanes solution (1:9) gives the title product as an oil (6.2 g, 60%).

Using essentially the same procedure but substituting 4-fluorophenyl cyclopropyl ketone for 4-chlorophenyl cyclopropyl ketone, 1-[1-(*p*-fluorophenyl)-2-methoxyvinyl]-cyclopropane, (E)- and (Z)- is obtained as an oil.

### EXAMPLE 15

### Preparation of p-Chloro-β-cyclopropyl-α-fluorocinnamaldehyde

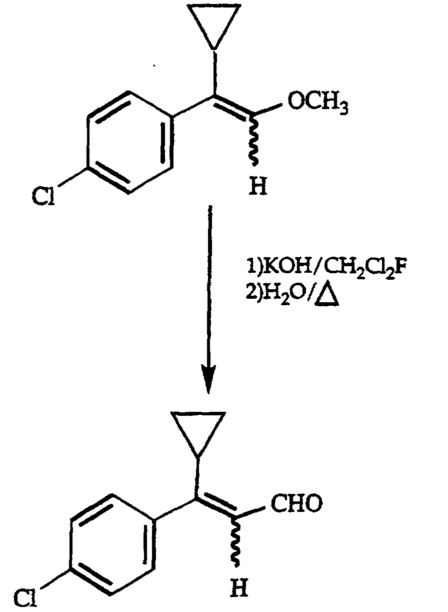

A mixture of potassium hydroxide (3.37 g, 0.060 mol), 18-Crown-6 (0.087 g, 0.33 mmol) and 1-[1-(*p*-chlorophenyl)- 2-methoxyvinyl]cyclopropane, (E)- and (Z)- (3.13 g, 0.015 mol) in water is treated with dichlorofluoromethane (8 g, 0.077 mol) at 7-10 °C, stirred at 10-13 °C overnight, treated with additional dichlorofluoromethane (6 g, 0.058 mol) at 7-10 °C, stirred at 10-13 °C for 36 hours, treated with water, stirred at 70-75 °C for 4 hours, cooled to room temperature, and extracted with ethyl acetate. The organic extracts are combined, washed sequentially with water, 2 N hydrochloric acid and water, dried over anhydrous sodium sulfate, and concentrated *in vacuo* to obtain a residue. Flash column chromatography of the residue using silica gel and an ethyl acetate/hexanes solution (1:9) gives 1.02 g of the E-isomer of the title product and 0.69 g of the Z- isomer of the title product (1.71 g total product).
Using essentially the same procedure, but substituting 1-[1-(*p*-fluorophenyl)-2-methoxyvinyl]cyclopropane,(E) - and (Z)- for 1-[1-(*p*-chlorophenyl)-2 methoxyvinyl]cyclopropane, (E) - and (Z) -, β-cyclopropyl-*p*,α-difluorocinnamaldehyde is obtained as an oil.

### EXAMPLE 16

### Preparation of (4-Fluoro-3-phenoxybenzyl)triphenyl phosphonium bromide

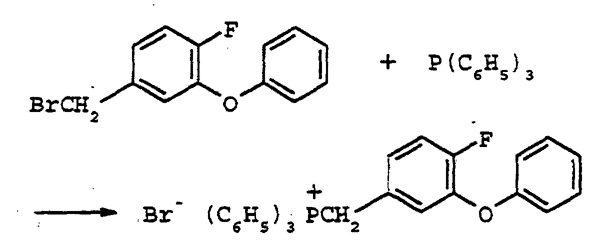

A solution of 4-fluoro-3-phenoxybenzyl bromide (42.17 g, 0.150 mol) in toluene is added to a solution of triphenyl phosphine (41.31 g, 0.158 mol) in toluene. The resultant reaction mixture is refluxed for one hour, cooled to room temperature, and filtered to obtain a solid. The solid is washed sequentially with toluene and hexanes, and dried in a dessicator at 60 °C to give the title product (73.7 g, 90.4%) which is identified by NMR spectral analyses.

Using essentially the same procedure, the following compounds are obtained:

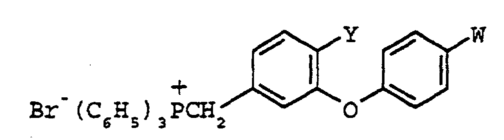

| **Y** | **W** |
|---|---|
| H | H |
| H | F |

### EXAMPLE 17

### Preparation of 1-(p-Chlorophenyl)-1-cyclopropyl-2-fluoro-4-(4-fluoro-3-phenoxyphenyl)-1,3-butadiene

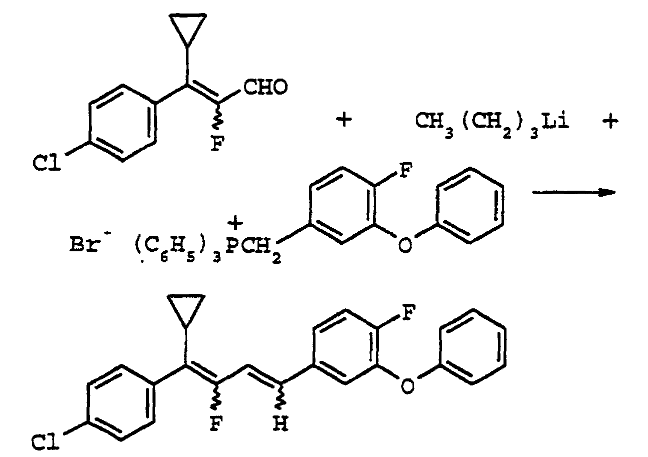

A mixture of (4-fluoro-3-phenoxybenzyl)triphenyl phosphonium bromide (41.77 g, 0.077 mol) in tetrahydrofuran is cooled to -55 to -60 °C, treated dropwise with a 2.5 M solution of butyllithium in hexanes (32.15 mL, 0.080 mol), warmed to and stirred at room temperature for 2 hours, cooled to -55 to -60 °C, treated dropwise with a solution of 2-fluoro-3-cyclopropyl-3-(*p*-chlorophenyl)acrylaldehyde (15.7 g, 0.070 mol) in tetrahydrofuran, warmed to and stirred at room temperature overnight, and quenched with ethyl acetate and 2 N hydrochloric acid. The resultant aqueous mixture is extracted with ethyl acetate. The organic extracts are combined, washed sequentially with water, 2 N hydrochloric acid and water, dried over anhydrous sodium sulfate, and concentrated *in vacuo* to obtain a residue. Flash column chromatography of the residue using silica gel and an ethyl acetate/hexanes solution (1:9) gives the title product as an oil (26.0 g, 91%).

Using essentially the same procedure, the following compounds are obtained:

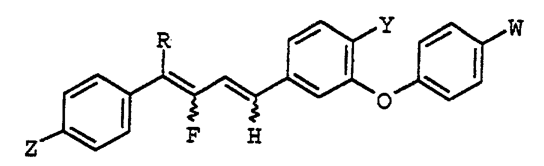

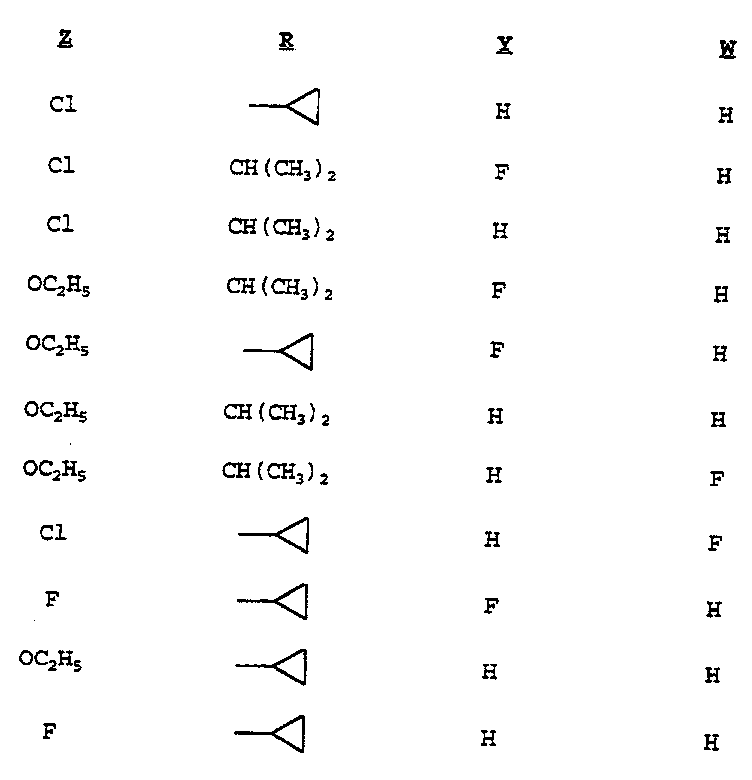

### EXAMPLE 18

### Preparation of 1-[1-(p-Chlorophenyl)-2-fluoro-4-(4-fluoro-3-phenoxyphenyl)-2-butenyl]cyclopropane, (R,S)-(Z)-

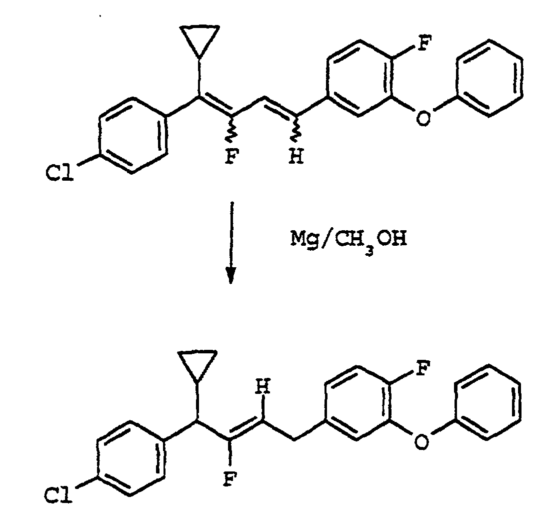

A solution of 1-(*p*-chlorophenyl)-1-cyclopropyl-2-fluoro-4-(4-fluoro-3-phenoxyphenyl)-1,3-butadiene (26 g, 0.064 mol) in a methanol/tetrahydrofuran solution (15:1) is treated with magnesium turnings (7.72 g, 0.317 mol), stirred at room temperature for 4 hours, quenched with hydrochloric acid, and extracted with ethyl acetate. The organic extracts are combined, washed sequentially with water, 2 N hydrochloric acid and water, dried over anhydrous sodium sulfate, and concentrated *in vacuo* to obtain a residue. Flash column chromatography of the residue using silica gel and an ethyl acetate/hexanes solution (5:95) gives the title product as an oil (21.4 g, 82%) which is identified by NMR spectral analyses.

Using essentially the same procedure, the following compounds are obtained:

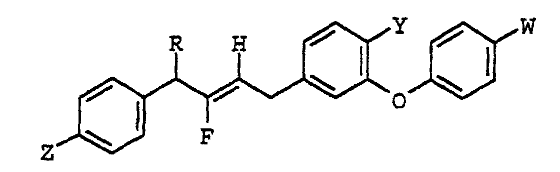

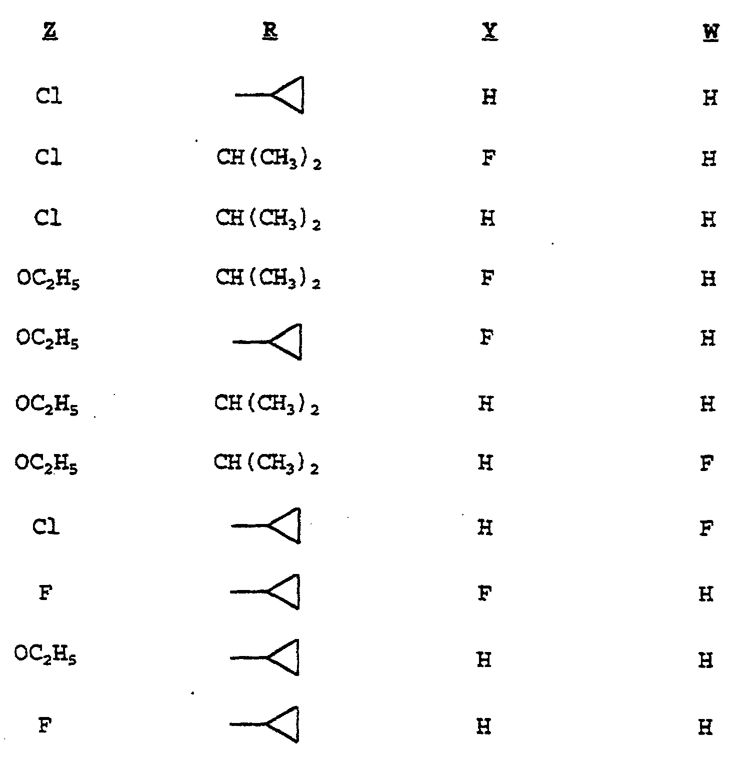

### EXAMPLE 19

### Insecticidal and acaricidal evaluation of test compounds

Test solutions are prepared by dissolving the test compound in a 35% acetone in water mixture to give a concentration of 10,000 ppm. Subsequent dilutions are made with water as needed.

### Spodoptera eridania, 3rd instar larvae, southern armyworm (SAW)

A Sieva lima bean leaf expanded to 7-8 cm in length is dipped in the test solution with agitation for 3 seconds and allowed to dry in a hood. The leaf is then placed in a 100 x 10 mm petri dish containing a damp filter paper on the bottom and ten 3rd instar caterpillars. At 5 days, observations are made of mortality, reduced feeding, or any interference with normal molting.

### Diabrotica virgifera virgifera Leconte, 3rd instar western corn rootworm (WCR)

One cc of fine talc is placed in a 30 mL wide-mouth screw-top glass jar. One mL of the appropriate acetone test solution is pipetted onto the talc so as to provide 1.25 mg of active ingredient per jar. The jars are set under a gentle air flow until the acetone is evaporated. The dried talc is loosened, 1 cc of millet seed is added to serve as food for the insects and 25 mL of moist soil is added to each jar. The jar is capped and the contents thoroughly mixed mechanically. Following this, ten 3rd instar rootworms are added to each jar and the jars are loosely capped to allow air exchange for the larvae. The treatments are held for 5 days when mortality counts are made. Missing larvae are presumed dead, since they decompose rapidly and cannot be found. The concentrations of active ingredient used in this test correspond approximately to 50 kg/ha.

### Heliothis virenscens, 3rd instar tobacco budworm (TBW)

Cotton cotyledons are dipped in the test solution and allowed to dry in a hood. When dry, each is cut into quarters and ten sections are placed individually in 30 mL plastic medicine cups containing a 5 to 7 mm long piece of damp dental wick. One 3rd instar caterpillar is added to each cup and a cardboard lid placed on the cup. Treatments are maintained for 3 days before mortality counts and estimates of reduction in feeding damage are made.

### Aphis fabae, mixed instar, bean aphid (BA)

Pots containing single nasturtium plants (Tropaeolum sp.) about 5 cm tall are infested with about 100-200 aphids one day before the test. Each pot is sprayed with the test solution for 2 revolutions of a 4 rpm turntable in a hood. The spray is directed to give complete coverage of the plants and aphids. The sprayed pots are set on their sides on white trays and held for 2 days, following which mortality estimates are made.

### Tetranychus urticae (OP-resistant strain), 2-spotted spider mite (TSM)

Sieva lima bean plants with primary leaves expanded to 7-8 cm are selected and cut back to one plant per pot. A small piece is cut from an infested leaf taken from the main colony and placed on each leaf of the test plants. This is done about 2 hours before treatment to allow the mites to move over to the test plant to lay eggs. The size of the cut, infested leaf is varied to obtain about 100 mites per leaf. At the time of test treatment, the piece of leaf used to transfer the mites is removed and discarded. The newly-infested plants are dipped in the test solution for 3 seconds with agitation and set in the hood to dry. After 2 days, one leaf is removed and mortality counts are made.

The tests are rated according to the scale shown below and the data obtained are shown in Table I.

Compounds employed in the above-described evaluations are given a compound number and identified by name. Data in Table I are reported by compound number.

**Rating Scale**

| | |
|---|---|
| 0 = no effect | 5 = 56-65% kill |
| 1 = 10-25% kill | 6 = 66-75% kill |
| 2 = 26-35% kill | 7 = 76-85% kill |
| 3 = 36-45% kill | 8 = 86-99% kill |
| 4 = 46-55% kill | 9 = 100% kill |
| - = not tested | |

### COMPOUNDS EVALUATED AS INSECTICIDAL AND ACARICIDAL AGENTS

| **Compound Number** | |
|---|---|
| 1 | 4- (*p*-Chlorophenyl)-3-fluoro-4-methyl-1-(*m*-phenoxyphenyl)-2-pentene, (Z)- and (E) - (95:5) |
| 2 | 4- (*p*-Chlorophenyl)-3-fluoro-1-(4-fluoro-3-phenoxyphenyl)-4-methyl-2-pentene, (Z)- |
| * 3 | 1-(*p*-Chlorophenyl)-1-[1-fluoro-3-(*m*-phenoxyphenyl)propenyl)cyclopropane, (Z)- |
| * 4 | 1- (*p*-Chlorophenyl)-1-[1-fluoro-3-(4-fluoro-3-phenoxyphenyl)propenyl]cyclopropane, (Z) - |
| 5 | 4-(*p*-Chlorophenyl)-3-fluoro-4-methyl-1-(*p*-phenoxyphenyl)-2-pentene, (Z)- |
| 6 | 1-(3-Chloro-4-fluorophenyl)-4-(*p*-chlorophenyl)-3-fluoro-4-methyl-2-pentene, (Z)- |
| 7 | 4- (*p*-Chlorophenyl) -3-fluoro-1- (4-fluoro-*m*-tolyl)-4-methyl-2-pentene, (Z) - |
| 8 | 1- [1-(*p*-Chlorophenyl)-2-fluoro-4-(4-fluoro-3-phenoxyphenyl)-2-butenyl]cyclopropane, (R,S)-(Z)- |
| 9 | 1-[1-(*p*-Chlorophenyl)-2-fluoro-4-(*m*-phenoxyphenyl)-2-butenyl]cyclopropane, (R,S)-(Z)- |
| 10 | 4-(*p*-Chlorophenyl)-3-fluoro-1-(4-fluoro-3-phenoxyphenyl) -5-methyl-2-hexene, (R,S)-(Z)- |
| 11 | 4-(*p*-Chlorophenyl)-3-fluoro-5-methyl-1-(*m*-phenoxyphenyl)-2-hexene, (R,S)-(Z)- |
| 12 | 4- (*p*-Ethoxyphenyl)-3-fluoro-1-(4-fluoro-3-phenoxyphenyl)-5-methyl-2-hexene, (R,S)-(Z)- |
| 13 | 1-[1-(*p*-Ethoxyphenyl)-2-fluoro-4-(4-fluoro-3-phenoxyphenyl)-2-butenyl]cyclopropane, (R,S)-(Z)- |
| 14 | 4- (*p*-Ethoxyphenyl) -3-fluoro-5-methyl-1-(*m*-phenoxyphenyl)-2-hexene, (R,S)-(Z)- |
| 15 | 4-(*p*-Ethoxyphenyl)-3-fluoro-1-[*m*-(*p*-fluorophenoxy)phenyl]-5-methyl-2-hexene, (R,S)-(Z)- |
| 16 | 1-{1-(*p*-Chlorophenyl)-2-fluoro-4-[*m*-(*p*-fluorophenoxy)phenyl]-2-butenyl}cyclopropane, (R,S)-(Z)- |
| 17 | 1- [2-Fluoro-4-(4-fluoro-3-phenoxyphenyl)-1-(*p*-fluorophenyl)-2-butenyl] cyclopropane, (R,S)-(Z)- |
| 18 | 1- [1-(*p*-Ethoxyphenyl)-2-fluoro-4-(*m*-phenoxyphenyl)-2-butenyl]cyclopropane, (R,S)-(Z)- |
| 19 | 1-[2-Fluoro-1-(*p*-fluorophenyl)-4-(*m*-phenoxyphenyl)-2-butenyl]cyclopropane, (R,S)-(Z)- |

| | |
|---|---|
| * compound not according to the invention | |

**TABLE I**

| **Insecticidal And Acaricidal Evaluations** | | | | | | |
|---|---|---|---|---|---|---|
| **Compound Number** | **SAW** | **WCR** | **TBW** | **BA** | **TSM** | |
| | **(100 ppm)** | **(50 ppm)** | **(100 ppm)** | **(100 ppm)** | **(300 ppm)** | **(100 ppm)** |
| 1 | 9 | 9 | 9 | 5 | 8 | 0 |
| 2 | 9 | 9 | 9 | 9 | 0 | - |
| *3 | 9 | 9 | 9 | 9 | 0 | - |
| * 4 | 9 | 9 | 9 | 9 | 2 | - |
| 5 | 0 | 0 | 0 | 4 | 1 | 3 |
| 6 | 0 | 0 | 1 | 0 | 1 | 2 |
| 7 | 0 | 6 | 0 | 4 | - | 0 |
| 8 | 9 | 9 | 9 | 9 | - | 7 |
| 9 | 9 | 9 | 9 | 8 | - | 8 |
| 10 | 9 | 9 | 9 | 8 | 8 | 0 |
| 11 | 9 | 9 | 9 | 7 | 8 | 0 |
| 12 | 9 | 9 | 9 | 9 | - | 0 |
| 13 | 9 | 9 | 9 | 9 | - | 9 |
| 14 | 9 | 9 | 9 | 8 | - | 0 |
| 15 | 9 | 9 | 9 | 3 | - | 0 |
| 16 | 9 | 9 | 9 | 8 | - | 7 |
| 17 | 9 | 9 | 9 | 9 | - | 7 |
| 18 | 9 | 9 | 9 | 9 | - | 8 |
| 19 | 9 | 9 | 9 | 9 | - | 2 |

| | | | | | | |
|---|---|---|---|---|---|---|
| * compound not according to the invention | | | | | | |

## Claims

1. A compound of Formula 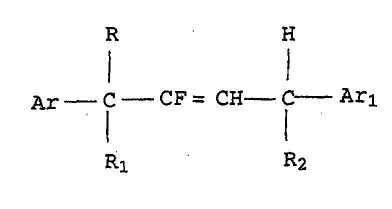 wherein
Ar ist phenyl optionally substituted with any combination of from one to three halogen, C₁-C₄alkyl, C₁-C₄haloalkyl, C₁-C₄alkoxy or C₁-C₄haloalkoxy groups,
1- or 2-naphthyl optionally substituted with any combination of from one to three halogen, C₁-C₄alkyl, C₁-C₄haloalkyl, C₁-C₄alkoxy or C₁-C₄haloalkoxy groups, or
a 5- or 6-membered heteroaromatic ring optionally substituted with any combination of from one to three halogen, C₁-C₄alkyl, C₁-C₄haloalkyl, C₁-C₄alkoxy or C₁-C₄haloalkoxy groups;
R and R₁ are each independently hydrogen, C₁-C₄alkyl, C₁-C₄haloalkyl, C₃-C₆cycloalkyl or C₃-C₆halocycloalkyl,
R₂ is hydrogen, Cl, Br, cyano or OR₃;
R₃ is hydrogen or C₁-C₄alkyl; and
Ar₁ is phenoxyphenyl optionally substituted with any combination of from one to six halogen, C₁-C₄alkyl, C₁-C₄haloalkyl, C₁-C₄alkoxy or C₁-C₄haloalkoxy groups,
phenyl optionally substituted with any combination of from one to five halogen, C₁-C₄alkyl, C₁-C₄haloalkyl, C₁-C₄alkoxy or C₁-C₄haloalkoxy groups,
biphenyl optionally substituted with any combination of from one to five halogen, C₁-C₄alkyl, C₁-C₄haloalkyl, C₁-C₄alkoxy or C₁-C₄haloalkoxy groups,
phenoxypyridyl optionally substituted with any combination of from one to five halogen, C₁-C₄alkyl, C₁-C₄haloalkyl, C₁-C₄alkoxy or C₁-C₄haloalkoxy groups,
benzylpyridyl optionally substituted with any combination of from one to five halogen, C₁-C₄alkyl, C₁-C₄haloalkyl, C₁-C₄alkoxy or C₁-C₄haloalkoxy groups,
benzylphenyl optionally substituted with any combination of from one to five halogen, C₁-C₄alkyl, C₁-C₄haloalkyl, C₁-C₄alkoxy or C₁-C₄haloalkoxy groups,
benzoylphenyl optionally substituted with any combination of from one to five halogen, C₁-C₄alkyl, C₁-C₄haloalkyl, C₁-C₄alkoxy or C₁-C₄haloalkoxy groups,
1- or 2-naphthyl optionally substituted with any combination of from one to three halogen, C₁-C₄alkyl, C₁-C₄haloalkyl, C₁-C₄alkoxy or C₁-C₄haloalkoxy groups, or
a 5- or 6-membered heteroaromatic ring optionally substituted with any combination of from one to three halogen, C₁-C₄alkyl, C₁-C₄haloalkyl, C₁-C₄alkoxy or C₁-C₄haloalkoxy groups, and
the optical isomers thereof, and
the cis and trans isomers thereof.

2. The compound of Formula I according to claim 1 wherein
Ar is phenyl optionally substituted with any combination of from one to three halogen, C₁-C₄alkyl, C₁-C₄haloalkyl, C₁-C₄alkoxy or C₁-C₄haloalkoxy groups;
R and R₁ are each independently hydrogen, C₁-C₄alkyl, C₁-C₄haloalkyl, C₃-C₆cycloalkyl or C₃-C₆halocycloalkyl provided that at least one of R and R₁ is other than hydrogen; and
Ar₁ is 3-phenoxyphenyl optionally substituted with any combination of from one to six halogen, C₁-C₄alkyl, C₁-C₄haloalkyl, C₁-C₄alkoxy or C₁-C₄haloalkoxy groups,
3-biphenyl optionally substituted with any combination of from one to five halogene, C₁-C₄alkyl, C₁-C₄haloalkyl, C₁-C₄alkoxy or C₁-C₄haloalkoxy groups, or
3-benzylphenyl optionally substituted with any combination of from one to five halogen, C₁-C₄alkyl, C₁-C₄haloalkyl, C₁-C₄alkoxy or C₁-C₄haloalkoxy groups.

3. The compound of Formula I according to claim 1 or claim 2
wherein
R is isopropyl or cyclopropyl and R₁ is hydrogen, or R and R₁ are methyl;
R₂ is hydrogen; and
Ar₁ is 3-phenyloxyphenyl optionally substituted with any combination of from one to six halogen, C₁-C₄alkyl, C₁-C₄haloalkyl, C₁-C₄-alkoxy or C₁-C₄haloalkoxy groups.

4. The compound of Formula I according to any one of claims 1 to 3 wherein
R is cyclopropyl and R₁ is hydrogen.

5. The compound of Formula I according to claim 1 selected from the group consisting of
1-[1-(*p*-chlorophenyl)-2-fluoro-4-(4-fluoro-3-phenoxyphenyl)-2-butenyl]cyclopropane, (R,S)-(Z)-;
1-[1-(*p*-chlorophenyl)-2-fluoro-4-(*m*-phenoxyphenyl)-2-butenyl]cyclopropane, (R,S)-(Z)-;
4-(*p*-chlorophenyl)-3-fluoro-1-(4-fluoro-3-phenoxyphenyl)-5-methyl-2-hexene, (R,S)-(Z)-;
4-(*p*-chlorophenyl)-3-fluoro-5-methyl-1-(*m*-phenoxyphenyl)-2-hexene, (R,S)-(Z)-;
4-(*p*-ethoxyphenyl)-3-fluoro-1-(4-fluoro-3-phenoxyphenyl)-5-methyl-2-hexene, (R,S)-(Z)-;
1-[1-(*p*-ethoxyphenyl)-2-fluoro-4-(4-fluoro-3-phenoxyphenyl)-2-butenyl]cyclopropane, (R,S)-(Z)-;
4-(*p*-ethoxyphenyl)-3-fluoro-5-methyl-1-(*m*-phenoxyphenyl)-2-hexene, (R,S)-(Z)-;
4-(*p*-ethoxyphenyl)-3-fluoro-1-[*m*-(*p*-fluorophenoxy)phenyl]-5-methyl-2-hexene, (R,S)-(Z)-;
1-{1-(*p*-chlorophenyl)-2-fluoro-4-[*m*-(*p*-fluorophenoxy)-phenyl]-2-butenyl}cyclopropane, (R,S)-(Z)-;
1-[2-fluoro-4-(4-fluoro-3-phenoxyphenyl)-1-(*p*-fluorophenyl)-2-butenyl]cyclopropane, (R,S)-(Z)-;
1-[1-(*p*-ethoxyphenyl)-2-fluoro-4-(*m*-phenoxyphenyl)-2-butenyl]cyclopropane, (R,S)-(Z)-;
1-[2-fluoro-1-(*p*-fluorophenyl)-4-(*m*-phenoxyphenyl)-2-butenyl]cyclopropane, (R,S)-(Z) ;
4-(*p*-chlorophenyl)-3-fluoro-4-methyl-1-(*m*-phenoxyphenyl)-2-pentene, (Z)-;
4-(*p*-chlorophenyl)-3-fluoro-1-(4-fluoro-3-phenoxyphenyl)-4-methyl-2-pentene, (Z)-;
1-(*p*-chlorophenyl)-1-[1-fluoro-3-(*m*-phenoxyphenyl)-propenyl]cyclopropane, (Z)-; and
1-(*p*-chlorophenyl)-1-[1-fluoro-3-(4-fluoro-3-phenoxyphenyl)propenyl]cyclopropane, (Z)-.

6. A method for the control of insect or acarid pests which comprises contacting said pests or their food supply, habitat or breeding grounds with a pesticidally effective amount of a compound of Formula I as claimed in any one of claims 1 to 5.

7. A method for the protection of growing plants from attack or infestation by insect or acarid pests which comprises applying to the foliage of the plants, or to the soil or water in which they are growing, a pesticidally effective amount of a compound of Formula I as claimed in any one of claims 1 to 5.

8. The method according to claim 7 wherein the compound of Formula I is applied to the plants, or to the soil or water in which they are growing, at a rate of 0.1 kg/ha to 4.0 kg/ha.

9. A composition for the control of insect or acarid pests which comprises an agronomically acceptable carrier and a pesticidally effective amount of a compound of Formula I as claimed in any one of claims 1 to 5.

10. A process for the preparation of a compound of Formula I as defined in claim 1 which comprises one of the following:
a) reacting a 4-aryl-1-bromo-3-fluoro-2-butene of Formula IX 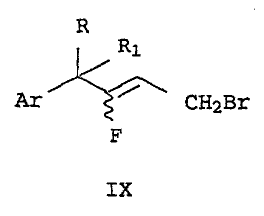 wherein Ar, R and R₁ are as defined in claim 1, with a base and a boronic acid having the structural formula (HO)₂BAr₁, wherein Ar₁ is as defined in claim 1, to provide a compound of Formula I wherein R₂ is H,
or b) reacting a diene of Formula XVII 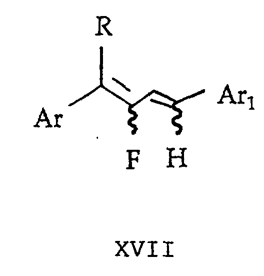 wherein Ar, R and Ar₁ are as defined in Claim 1, with magnesium in the presence of a protic solvent to provide the corresponding compound of Formula I wherein R₁ and R₂ are both H,
or c) halogenating a compound of Formula I wherein R₂ is H with a chlorinating agent or a brominating agent to provide the corresponding compound of Formula I wherein R₂ is Cl or Br,
or d) reacting a compound of Formula I wherein R₂ is Cl or Br with sodium cyanide to provide the corresponding compound of Formula I wherein R₂ is CN,
or e) reacting a compound of Formula I wherein R₂ is Cl or Br with sodium hydroxide to provide the corresponding compound of Formula I wherein R₂ is OH,
or f) reacting a compound of Formula I wherein R₂ is Cl or Br with a compound of formula NaOR₄, wherein R₄ is a C₁-C₄ alkyl group, to provide the corresponding compound of Formula I wherein R₂ is a C₁-C₄ alkoxy group,
or g) reacting a compound of Formula I wherein R₂ is OH with a C₁-C₄ alkyl halide to provide the corresponding compound of Formula I wherein R₂ is a C₁-C₄ alkoxy group,
or h) reacting a compound of formula Br-Ar₁, wherein Ar₁ is as defined in claim 1, with a compound of the following formula: 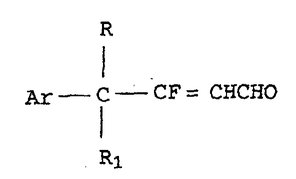 wherein Ar, R and R₁ are as defined in claim 1, to provide a compound of Formula I wherein R₂ is OH,
or i) reacting a compound of Formula I wherein R₂ is OH with a thionyl chloride or thionyl bromide to provide the corresponding compound of Formula I wherein R₂ is Cl or Br.

11. A process for the preparation of a compound of Formula I as claimed in any one of claims 1 to 5 which process comprises reacting a 4-aryl-3-fluoro-2-buten-1-ol of Formula VIII 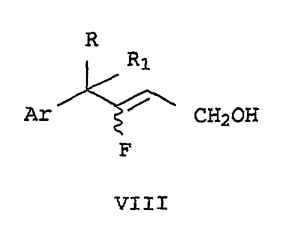 wherein Ar, R and R₁ are as defined in claim 1 with a brominating agent to form a 4-aryl-1-bromo-3-fluoro-2-butene of Formula XI 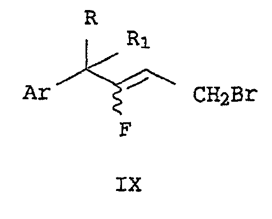 and reacting the 4-aryl-1-bromo-3-fluoro-2-butene with 0.0025 to 0.1 molar equivalent of a palladium catalyst, at least 2 molar equivalents of a base and a boronic acid having the structural formula
(HO)₂BAr₁
wherein Ar₁ is as defined in claim 1.

12. A process for the preparation of a compound of Formula I as claimed in any one of claims 1 to 5 which process comprises reacting a 3-aryl-2-fluoro-2-propenal of Formula XV 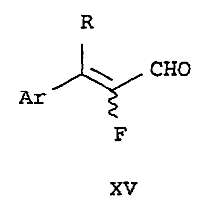 wherein Ar and R are as defined in claim 1, with an ylide of Formula XVI 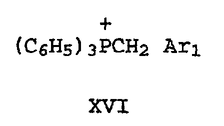 wherein Ar₁ is as defined in claim 1 in the presence of a base to form a diene having the structural formula 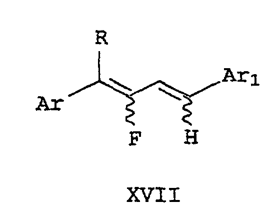 wherein Ar, R and Ar₁ are as defined in claim 1, and reacting the diene with magnesium in the presence of a protic solvent.

13. A compound of Formula XXX 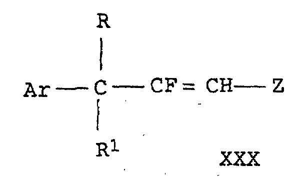 wherein Ar, R and R₁ are as defined in claim 1 and Z is cyano, CHO, CH₂OH or CH₂Br,
and the optical isomers thereof, and the cis and trans isomers thereof.

14. The compound of Formula XXX according to claim 13 wherein
Ar is phenyl optionally substituted with any combination of from one to three halogen, C₁-C₄alkyl, C₁-C₄haloalkyl, C₁-C₄alkoxy or C₁-C₄haloalkoxy groups.

15. The compound of Formula XXX according to claim 13 or Claim 14
wherein
R and R₁ are methyl.

16. A process for the preparation of a compound of Formula XXX as defined in any one of claims 13 to 15 which comprises one of the following:
a) reacting a compound of Formula V 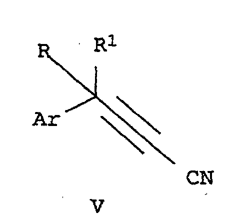 wherein Ar, R and R₁ are as defined in claim 1 with caesium fluoride, potassium hydrogen fluoride and water in N,N-dimethylformamide to provide a compound of Formula XXX
wherein Z = CN,
or b) selectively reducing a compound of Formula XXX wherein Z = CN to provide the corresponding compound of formula XXX wherein Z = CHO,
or c) reducing a compound of formula XXX wherein Z = CHO to provide the corresponding compound of formula XXX wherein Z = CH₂OH,
or d) reacting compound of formula XXX wherein Z = CH₂OH with a brominating agent to provide the corresponding compound of formula XXX wherein Z= CH₂Br.

17. A compound of Formula XXXI 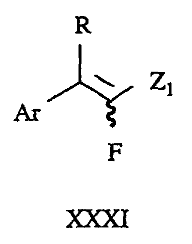 wherein
wherein Ar and R are as defined in Claim 1 and
Z₁ is C₁-C₄alkoxycarbonyl, CH₂OH, CHO, CH₂Cl or CH=CHAr₁; and and Ar₁ is as defined in Claim 1,
and the optical isomers thereof, and the cis and trans isomers thereof.

18. The compound of Formula XXXI according to claim 17
wherein
Ar is phenyl optionally substituted with any combination of from one to three halogen, C₁-C₄alkyl, C₁-C₄haloalkyl, C₁-C₄alkoxy or C₁-C₄haloalkoxy groups; and
Ar₁ is 3-phenoxyphenyl optionally substituted with any combination of from one to six halogen, C₁-C₄alkyl, C₁-C₄haloalkyl, C₁-C₄alkoxy or C₁-C₄haloalkoxy groups.

19. The compound of Formula XXXI according to Claim 17 or Claim 18 wherein
R is isopropyl or cyclopropyl.

20. A process for the preparation of a compound of Formula XXXI as defined in any one of Claims 17 to 19 which comprises one of the following:
a) reacting a ketone or aldehyde of formula XI 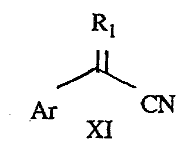 with an ester of formula XII 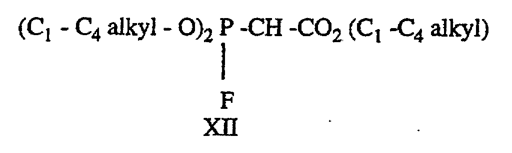 to provide a compound of Formula XXXI wherein Z₁= a C₁-C₄ alkyloxycarbonyl group,
or b) reducing a compound of Formula XXXI wherein Z₁= a C₁-C₄ alkyloxycarbonyl group to provide the corresponding compound of Formula XXXI wherein Z₁= CH₂OH,
or c) oxidizing a compound of Formula XXXI wherein Z₁= CH₂OH to provide the corresponding compound of Formula XXXI wherein Z₁= CHO,
or d) reacting a compound of Formula XXXI wherein Z₁= CHO with an ylide of formula XVI 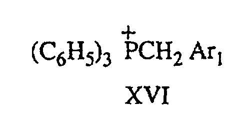 to provide a diene of formula XVII 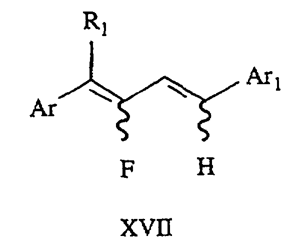
or e) reacting a compound of formula XIV 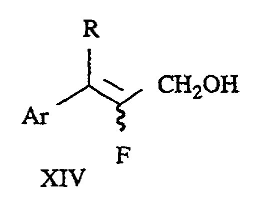 with thionyl chloride to provide the corresponding compound of Formula XXXI wherein Z₁= CH₂Cl,
or f) reacting an ylide of formula XIX 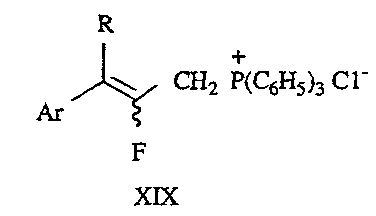 with an aldehyde of formula XX 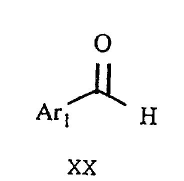 to provide a diene of formula XVII 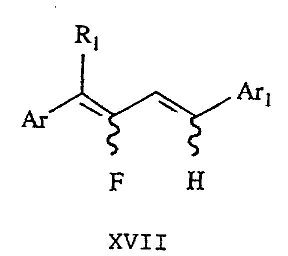
or g) reacting a compound of formula XXII 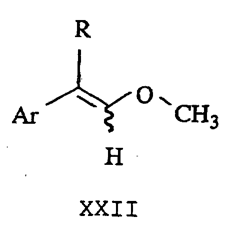 with dichlorofluoromethane to provide a compound of Formula XXXI wherein Z₁=CHO.

## Patentansprüche

1. Verbindung der Formel 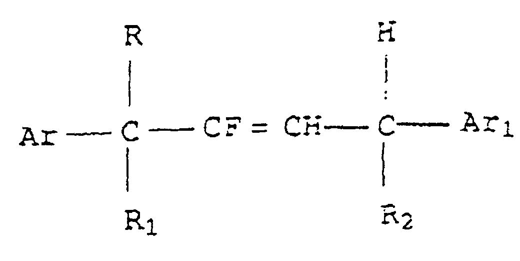 in der
Ar Phenyl, das gegebenenfalls mit einer beliebigen Kombination von ein bis drei Halogen-, C₁-C₄Alkyl-, C₁-C₄Halogenalkyl-, C₁-C₄Alkoxy- oder C₁-C₄Halogenalkoxygruppen substituiert ist,
1- oder 2-Naphthyl, das gegebenenfalls mit einer beliebigen Kombination von ein bis drei Halogen-, C₁-C₄Alkyl-, C₁-C₄Halogenalkyl-, C₁-C₄Alkoxy- oder C₁-C₄Halogenalkoxygruppen substituiert ist, oder
einen 5- oder 6-gliedrigen heteroaromatischen Ring, der gegebenenfalls mit einer beliebigen Kombination von ein bis drei Halogen-, C₁-C₄Alkyl-, C₁-C₄Halogenalkyl-, C₁-C₄Alkoxy- oder C₁-C₄Halogenalkoxygruppen substituiert ist, bedeutet;
R und R₁ jeweils unabhängig voneinander Wasserstoff, C₁-C₄Alkyl, C₁-C₄Halogenalkyl, C₃-C₆Cycloalkyl oder C₃-C₆Halogencycloalkyl bedeuten,
R₂ Wasserstoff, Cl, Br, Cyano oder OR₃ bedeutet;
R₃ Wasserstoff oder C₁-C₄Alkyl bedeutet; und
Ar₁ Phenoxyphenyl, das gegebenenfalls mit einer beliebigen Kombination von ein bis sechs Halogen-, C₁-C₄Alkyl-, C₁-C₄Halogenalkyl-, C₁-C₄Alkoxy- oder C₁-C₄Halogenalkoxygruppen substituiert ist,
Phenyl, das gegebenenfalls mit einer beliebigen Kombination von ein bis fünf Halogen-, C₁-C₄Alkyl-, C₁-C₄Halogenalkyl-, C₁-C₄Alkoxy- oder C₁-C₄Halogenalkoxygruppen substituiert ist,
Biphenyl, das gegebenenfalls mit einer beliebigen Kombination von ein bis fünf Halogen-, C₁-C₄Alkyl-, C₁-C₄Halogenalkyl-, C₁-C₄Alkoxy- oder C₁-C₄Halogenalkoxygruppen substituiert ist,
Phenoxypyridyl, das gegebenenfalls mit einer beliebigen Kombination von ein bis fünf Halogen-, C₁-C₄Alkyl-, C₁-C₄Halogenalkyl-, C₁-C₄Alkoxy- oder C₁-C₄Halogenalkoxygruppen substituiert ist,
Benzylpyridyl, das gegebenenfalls mit einer beliebigen Kombination von ein bis fünf Halogen-, C₁-C₄Alkyl-, C₁-C₄Halogenalkyl-, C₁-C₄Alkoxy- oder C₁-C₄Halogenalkoxygruppen substituiert ist,
Benzylphenyl, das gegebenenfalls mit einer beliebigen Kombination von ein bis fünf Halogen-, C₁-C₄Alkyl-, C₁-C₄Halogenalkyl-, C₁-C₄Alkoxy- oder C₁-C₄Halogenalkoxygruppen substituiert ist,
Benzoylphenyl, das gegebenenfalls mit einer beliebigen Kombination von ein bis fünf Halogen-, C₁-C₄Alkyl-, C₁-C₄Halogenalkyl-, C₁-C₄Alkoxy- oder C₁-C₄Halogenalkoxygruppen substituiert ist,
1- oder 2-Naphthyl, das gegebenenfalls mit einer beliebigen Kombination von ein bis drei Halogen-, C₁-C₄Alkyl-, C₁-C₄Halogenalkyl-, C₁-C₄Alkoxy- oder C₁-C₄Halogenalkoxygruppen substituiert ist, oder
einen 5- oder 6-gliedrigen heteroaromatischen Ring, der gegebenenfalls mit einer beliebigen Kombination von ein bis drei Halogen-, C₁-C₄Alkyl-, C₁-C₄Halogenalkyl-, C₁-C₄Alkoxy- oder C₁-C₄Halogenalkoxygruppen substituiert ist, bedeutet; sowie
deren optische Isomere und
deren cis- und trans-Isomere.

2. Verbindung der Formel I nach Anspruch 1, in der
Ar Phenyl, das gegebenenfalls mit einer beliebigen Kombination von ein bis drei Halogen-, C₁-C₄Alkyl-, C₁-C₄Halogenalkyl-, C₁-C₄Alkoxy- oder C₁-C₄Halogenalkoxygruppen substituiert ist,
R und R₁ jeweils unabhängig voneinander Wasserstoff, C₁-C₄Alkyl, C₁-C₄Halogenalkyl, C₃-C₆Cycloalkyl oder C₃-C₆Halogencycloalkyl bedeuten,
vorausgesetzt, daß zumindest einer der Substituenten R und R₁ nicht Wasserstoff bedeutet, und
Ar₁ 3-Phenoxyphenyl, das gegebenenfalls mit einer beliebigen Kombination von ein bis sechs Halogen-, C₁-C₄Alkyl-, C₁-C₄Halogenalkyl-, C₁-C₄Alkoxy- oder C₁-C₄Halogenalkoxygruppen substituiert ist,
3-Biphenyl, das gegebenenfalls mit einer beliebigen Kombination von ein bis fünf Halogen-, C₁-C₄Alkyl-, C₁-C₄Halogenalkyl-, C₁-C₄Alkoxy- oder C₁-C₄Halogenalkoxygruppen substituiert ist,
3-Benzylphenyl, das gegebenenfalls mit einer beliebigen Kombination von ein bis fünf Halogen-, C₁-C₄Alkyl-, C₁-C₄Halogenalkyl-, C₁-C₄Alkoxy- oder C₁-C₄Halogenalkoxygruppen substituiert ist, bedeutet.

3. Verbindung der Formel I nach Anspruch 1 oder 2, in der
R Isopropyl oder Cyclopropyl bedeutet und R₁ Wasserstoff bedeutet, oder R und R₁ Methyl bedeuten;
R₂ Wasserstoff bedeutet; und
Ar₁ 3-Phenoxyphenyl, das gegebenenfalls mit einer beliebigen Kombination von ein bis sechs Halogen-, C₁-C₄Alkyl-, C₁-C₄Halogenalkyl-, C₁-C₄Alkoxy- oder C₁-C₄Halogenalkoxygruppen substituiert ist, bedeutet.

4. Verbindung der Formel I nach einem der Ansprüche 1 bis 3, in der
R Cyclopropyl bedeutet und R₁ Wasserstoff bedeutet.

5. Verbindung der Formel I nach Anspruch 1 aus der Gruppe
1-[1-(*p*-Chlorphenyl)-2-fluor-4-(4-fluor-3-phenoxyphenyl)-2-butenyl]cyclopropan, (R,S)-(Z)-;
1-[1-(*p*-Chlorphenyl)-2-fluor-4-(*m*-phenoxyphenyl)-2-butenyl]cyclopropan, (R,S)-(Z)-;
4-(*p*-Chlorphenyl)-3-fluor-1-(4-fluor-3-phenoxyphenyl)-5-methyl-2-hexen, (R,S)-(Z)-;
4-(*p*-Chlorphenyl)-3-fluor-5-methyl-1-(*m*-phenoxyphenyl)-2-hexen, (R,S)-(Z)-;
4-(*p*-Ethoxyphenyl)-3-fluor-1-(4-fluor-3-phenoxyphenyl)-5-methyl-2-hexen, (R,S)-(Z)-;
1-[1-(*p*-Ethoxyphenyl)-2-fluor-4-(4-fluor-3-phenoxyphenyl)-2-butenyl] cyclopropan, (R,S)-(Z)-;
4-(*p*-Ethoxyphenyl)-3-fluor-5-methyl-1-(*m*-phenoxyphenyl)-2-hexen, (R,S)-(Z)-;
4-(*p*-Ethoxyphenyl)-3-fluor-1-[*m-*(p-fluorphenoxy)phenyl]-5-methyl-2-hexen, (R,S)-(Z)-;
1-{1-(*p*-Chlorphenyl)-2-fluor-4-[*m*-(p-fluorphenoxy)phenyl]-2-butenyl}cyclopropan,(R,S)-(Z)-;
1-[2-Fluor-4-(4-fluor-3-phenoxyphenyl)-1-(*p*-fluorphenyl)-2-butenyl]cyclopropan, (R,S)-(Z)-;
1-[1-(*p*-Ethoxyphenyl)-2-fluor-4-(*m*-phenoxyphenyl)-2-butenyl]cyclopropan, (R,S)-(Z)-;
1-[2-Fluor-1-(*p*-fluorphenyl)-4-(*m*-phenoxyphenyl)-2-butenyl]cyclopropan, (R,S)-(Z);
4-(*p*-Chlorphenyl)-3-fluor-4-methyl-1-(*m*-phenoxyphenyl)-2-penten, (Z)-;
4-(*p*-Chlorphenyl)-3-fluor-1-(4-fluor-3-phenoxyphenyl)-4-methyl-2-penten, (Z)-;
1-(*p*-Chlorphenyl)-1-[1-fluor-3-(*m*-phenoxyphenyl)-propenyl]cyclopropan, (Z)-; und
1-(*p*-Chlorphenyl)-1-[1-fluor-3-(4-fluor-3-phenoxyphenyl)propenyl]cyclopropan, (Z)-.

6. Verfahren zur Bekämpfung von Schadinsekten oder Schadakariden, **dadurch gekennzeichnet, daß** man diese Schädlinge oder ihre Nahrungsversorgung, Umwelt oder Brutstätten mit einer pestizid wirksamen Menge einer Verbindung der Formel I nach einem der Ansprüche 1 bis 5 in Kontakt bringt.

7. Verfahren zum Schutz wachsender Pflanzen gegen den Angriff oder Befall durch Schadinsekten oder Schadakariden, **dadurch gekennzeichnet, daß** man eine pestizid wirksame Menge einer Verbindung der Formel I nach einem der Ansprüche 1 bis 5 auf das Blattwerk der Pflanzen oder den Boden oder das Wasser, in dem sie wachsen, aufbringt.

8. Verfahren nach Anspruch 7, wobei die Verbindung der Formel I auf die Pflanzen oder auf den Boden oder das Wasser, in dem sie wachsen, in einer Menge von 0,1 kg/ha bis 4,0 kg/ha aufgebracht wird.

9. Zusammensetzung zur Bekämpfung von Schadinsekten oder Schadakariden mit einem landwirtschaftlich unbedenklichen Träger und einer pestizid wirksamen Menge einer Verbindung der Formel I nach einem der Ansprüche 1 bis 5.

10. Verfahren zur Herstellung einer Verbindung der Formel I nach Anspruch 1, **dadurch gekennzeichnet, daß** man entweder
a) ein 4-Aryl-1-brom-3-fluor-2-buten der Formel IX 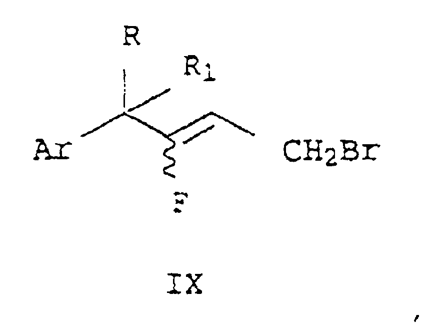 in der Ar, R und R₁ wie in Anspruch 1 definiert sind, mit einer Base und einer Boronsäure der Strukturformel (HO)₂BAr₁, in der Ar₁ wie in Anspruch 1 definiert ist, umsetzt, wodurch man eine Verbindung der Formel I, in der R₂ H bedeutet, erhält
oder b) ein Dien der Formel XVII 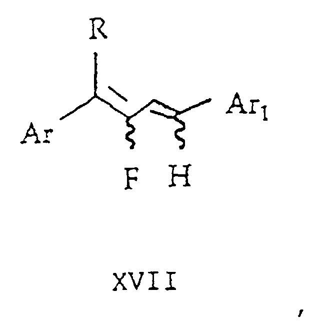 in der Ar, R und Ar₁ wie in Anspruch 1 definiert ist, mit Magnesium in Gegenwart eines protischen Lösungsmittels umsetzt, wodurch man die entsprechende Verbindung der Formel I, in der R₁ und R₂ beide H bedeuten, erhält
oder c) eine Verbindung der Formel I, in der R₂ H bedeutet, mit einem Chlorierungsmittel oder einem Bromierungsmittel halogeniert, wodurch man die entsprechende Verbindung der Formel I, in der R₂ Cl oder Br bedeutet, erhält
oder d) eine Verbindung der Formel I, in der R₂ Cl oder Br bedeutet, mit Natriumcyanid umsetzt, wodurch man die entsprechende Verbindung der Formel I, in der R₂ CN bedeutet, erhält
oder e) eine Verbindung der Formel I, in der R₂ Cl oder Br bedeutet, mit Natriumhydroxid umsetzt, wodurch man die entsprechende Verbindung der Formel I, in der R₂ OH bedeutet, erhält
oder f) eine Verbindung der Formel I, in der R₂ Cl oder Br bedeutet, mit einer Verbindung der Formel NaOR₄, in der R₄ eine C₁-C₄Alkylgruppe bedeutet, umsetzt, wodurch man die entsprechende Verbindung der Formel I, in der R₂ eine C₁-C₄Alkoxygruppe bedeutet, erhält
oder g) eine Verbindung der Formel I, in der R₂ OH bedeutet, mit einem C₁-C₄Alkylhalogenid umsetzt, wodurch man die entsprechende Verbindung der Formel I gelangt, in der R₂ eine C₁-C₄Alkoxygruppe bedeutet, erhält
oder h) eine Verbindung der Formel Br-Ar₁, in der Ar₁ wie in Anspruch 1 definiert ist, mit einer Verbindung der Formel 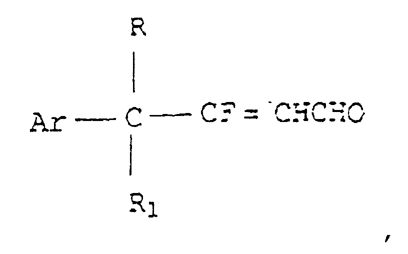 in der Ar, R und R₁ wie in Anspruch 1 definiert sind, umsetzt, wodurch man eine Verbindung der Formel I, in der R₂ OH bedeutet, erhält
oder i) eine Verbindung der Formel I, in der R₂ OH bedeutet, mit einem Thionylchlorid oder Thionylbromid umsetzt, wodurch man die entsprechende Verbindung der Formel I, in der R₂ Cl oder Br bedeutet, erhält.

11. Verfahren zur Herstellung einer Verbindung der Formel I nach einem der Ansprüche 1 bis 5, **dadurch gekennzeichnet, daß** man ein 4-Aryl-3-fluor-2-buten-1-ol der Formel VIII 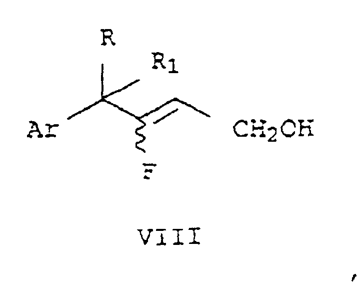 in der Ar, R und R₁ wie in Anspruch 1 definiert sind, mit einem Bromierungsmittel umsetzt, wodurch man ein 4-Aryl-1-brom-3-fluor-2-buten der Formel XI 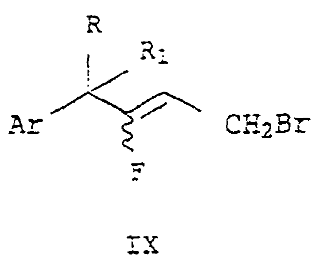 erhält und das 4-Aryl-1-brom-3-fluor-2-buten mit 0,0025 bis 0,1 Moläquivalent eines Palladiumkatalysators, mindestens 2 Moläquivalenten einer Base und einer Boronsäure mit der Strukturformel
(HO)₂BAr₁,
in der Ar₁ wie in Anspruch 1 definiert ist, umsetzt.

12. Verfahren zur Herstellung einer Verbindung der Formel I nach einem der Ansprüche 1 bis 5, **dadurch gekennzeichnet, daß** man ein 3-Aryl-2-fluor-2-propenal der Formel XV 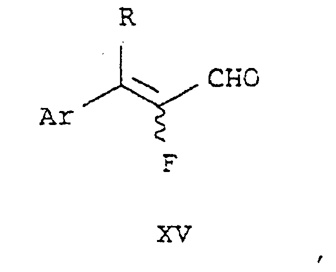 in der Ar und R wie in Anspruch 1 definiert sind, mit einem Ylid der Formel XVI 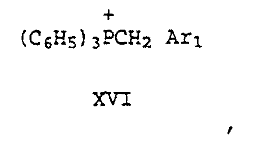 in der Ar₁ wie in Anspruch 1 definiert ist, in Gegenwart einer Base umsetzt, wodurch man ein Dien mit der Strukturformel 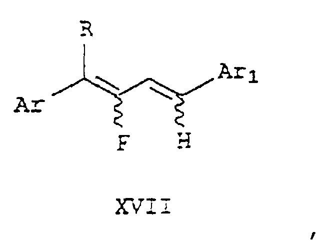 in der Ar, R und Ar₁ wie in Anspruch 1 definiert sind, erhält, und das Dien mit Magnesium in Gegenwart eines protischen Lösungsmittels umsetzt.

13. Verbindung der Formel XXX 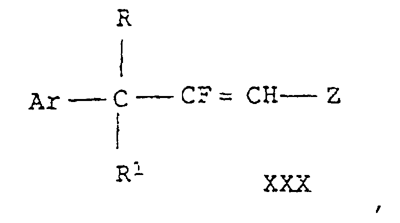 in der Ar, R und R₁ wie in Anspruch 1 definiert sind und
Z Cyan, CHO, CH₂OH oder CH₂Br bedeutet,
und deren optische Isomere und deren cis- und trans-Isomere.

14. Verbindung der Formel XXX nach Anspruch 13, in der
Ar Phenyl bedeutet, das gegebenenfalls mit einer beliebigen Kombination von ein bis drei Halogen-, C₁-C₄Alkyl-, C₁-C₄Halogenalkyl-, C₁-C₄Alkoxy- oder C₁-C₄Halogenalkoxygruppen substituiert ist, bedeutet.

15. Verbindung der Formel XXX nach Anspruch 13 oder Anspruch 14, in der
R und R₁ Methyl bedeuten.

16. Verfahren zur Herstellung einer Verbindung der Formel XXX nach einem der Ansprüche 13 bis 15, **dadurch gekennzeichnet, daß** man entweder
a) eine Verbindung der Formel V 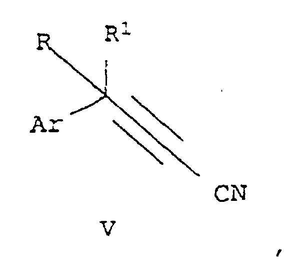 in der Ar, R und R₁, wie in Anspruch 1 definiert sind, mit Cäsiumfluorid, Kaliumhydrogenfluorid und Wasser in N,N-Dimethylformamid umsetzt, wodurch man eine Verbindung der Formel XXX, in der Z = CN bedeutet, erhält
oder b) eine Verbindung der Formel XXX, in der Z = CN bedeutet, selektiv reduziert, wodurch man die entsprechende Verbindung der Formel XXX, in der Z = CHO bedeutet, erhält
oder c) eine Verbindung der Formel XXX, in der Z CHO bedeutet, reduziert, wodurch man die entsprechende Verbindung der Formel XXX, in der Z = CH₂OH bedeutet, erhält
oder d) eine Verbindung der Formel XXX, in der Z CH₂OH bedeutet, mit einem Bromierungsmittel umsetzt, wodurch man die entsprechende Verbindung der Formel XXX, in der Z = CH₂Br bedeutet, erhält.

17. Verbindung Formel XXXI 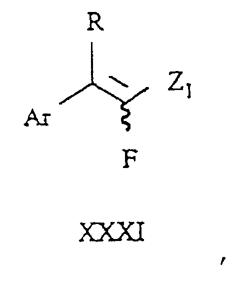 in der
Ar und R wie in Anspruch 1 definiert sind und
Z₁ C₁-C₄Alkoxycarbonyl, CH₂OH, CHO, CH₂Cl oder CH=CHAr₁ bedeutet; und
Ar₁ wie in Anspruch 1 definiert ist,
und ihre optischen Isomere und ihre cis- und trans-Isomere.

18. Verbindung der Formel XXXI nach Anspruch 17, in der
Ar Phenyl, das gegebenenfalls mit einer beliebigen Kombination von ein bis drei Halogen-, C₁-C₄Alkyl-, C₁-C₄Halogenalkyl-, C₁-C₄Alkoxy- oder C₁-C₄Halogenalkoxygruppen substituiert ist, bedeutet, und
Ar₁ 3-Phenoxyphenyl, das gegebenenfalls mit einer beliebigen Kombination von ein bis sechs Halogen-, C₁-C₄Alkyl-, C₁-C₄Halogenalkyl-, C₁-C₄Alkoxy- oder C₁-C₄Halogenalkoxygruppen substituiert ist, bedeutet.

19. Verbindung der Formel XXXI nach Anspruch 17 oder 18, in der R Isopropyl oder Cyclopropyl bedeutet.

20. Verfahren zur Herstellung einer Verbindung der Formel XXXI nach einem der Ansprüche 17 bis 19, **dadurch gekennzeichnet, daß** man entweder
a) ein Keton bzw. einen Aldehyd der Formel XI 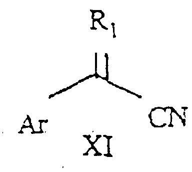 mit einem Ester der Formel XII 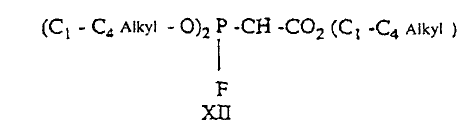 umsetzt, wodurch man eine Verbindung der Formel XXXI, in der Z₁ eine C₁-C₄Alkyloxycarbonylgruppe bedeutet, erhält
oder b) eine Verbindung der Formel XXXI, in der Z₁ eine C₁-C₄Alkyloxycarbonylgruppe bedeutet, reduziert, wodurch man die entsprechende Verbindung der Formel XXXI, in der Z₁= CH₂OH bedeutet, erhält
oder c) eine Verbindung der Formel XXXI, in der Z₁ CH₂OH bedeutet, oxidiert, wodurch man die entsprechende Verbindung der Formel XXXI, in der Z₁ CHO bedeutet, erhält
oder d) eine Verbindung der Formel XXXI, in der Z₁ CHO bedeutet, mit einem Ylid der Formel XVI 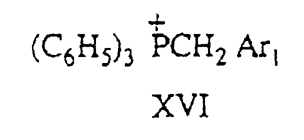 umsetzt, wodurch man zu einem Dien der Formel XVII 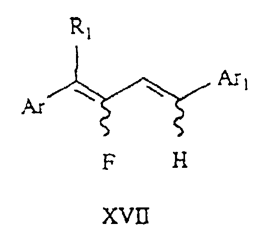 gelangt,
oder e) eine Verbindung der Formel XIV 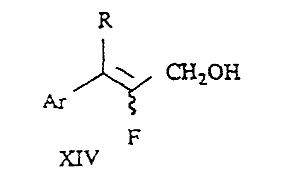 mit Thionylchlorid umsetzt, wodurch man die entsprechende Verbindung der Formel XXXI, in der Z₁ CH₂Cl bedeutet, erhält
oder f) ein Ylid der Formel XIX 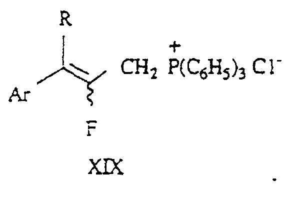 mit einem Aldehyd der Formel XX 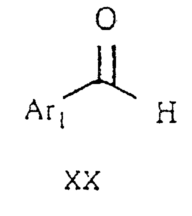 umsetzt, wodurch man ein Dien der Formel XVII 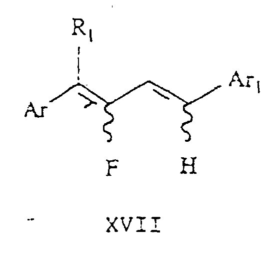 erhält,
oder g) eine Verbindung der Formel XXII 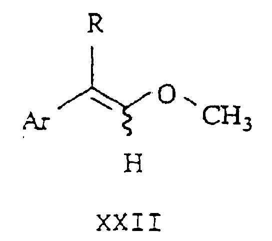 mit Dichlorfluormethan umsetzt, wodurch man eine Verbindung der Formel XXXI, in der Z₁ CHO bedeutet, erhält.

## Revendications

1. Composé de formule I 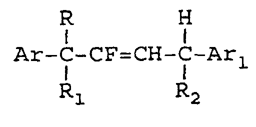 dans laquelle
Ar est un groupe phényle facultativement substitué par toute association de un à trois groupes halogéno, alkyle en C₁-C₄, halogénoalkyle en C₁-C₄, alcoxy en C₁-C₄, halogénoalcoxy en C₁-C₄,
1- ou 2-naphtyle facultativement substitué par toute association de un à trois groupes halogéno, alkyle en C₁-C₄, halogénoalkyle en C₁-C₄, alcoxy en C₁-C₄ ou halogénoalcoxy en C₁-C₄, ou
un cycle hétéroaromatique penta- ou hexagonal facultativement substitué par toute association de un à trois groupes halogéno, alkyle en C₁-C₄, halogénoalkyle en C₁-C₄, alcoxy en C₁-C₄ ou halogénoalcoxy en C₁-C₄ ;
R et R₁ sont chacun indépendamment l'hydrogène, un groupe alkyle en C₁-C₄, halogénoalkyle en C₁-C₄, cycloalkyle en C₃-C₆ ou halogénocycloalkyle en C₃-C₆ ;
R₂ est l'hydrogène, Cl, Br, cyano ou OR₃ ;
R₃ est l'hydrogène ou un groupe alkyle en C₁-C₄ ;
Ar₁ est un groupe phénoxyphényle facultativement substitué par toute association de un à six groupes halogéno, alkyle en C₁-C₄, halogénoalkyle en C₁-C₄, alcoxy en C₁-C₄ ou halogénoalcoxy en C₁-C₄,
phényle facultativement substitué par toute association de un à cinq groupes halogéno, alkyle en C₁-C₄, halogénoalkyle en C₁-C₄, alcoxy en C₁-C₄ ou halogénoalcoxy en C₁-C₄,
biphénylyle facultativement substitué par toute association de un à cinq groupes halogéno, alkyle en C₁-C₄, halogénoalkyle en C₁-C₄, alcoxy en C₁-C₄ ou halogénoalcoxy en C₁-C₄,
phénoxypyridyle facultativement substitué par toute association de un à cinq groupes halogéno, alkyle en C₁-C₄, halogénoalkyle en C₁-C₄, alcoxy en C₁-C₄ ou halogénoalcoxy en C₁-C₄,
benzylpyridyle facultativement substitué par toute association de un à cinq groupes halogéno, alkyle en C₁-C₄, halogénoalkyle en C₁-C₄, alcoxy en C₁-C₄ ou halogénoalcoxy en C₁-C₄,
benzylphényle facultativement substitué par toute association de un à cinq groupes halogéno, alkyle en C₁-C₄, halogénoalkyle en C₁-C₄, alcoxy en C₁-C₄ ou halogénoalcoxy en C₁-C₄,
benzoylphényle facultativement substitué par toute association de un à cinq groupes halogéno, alkyle en C₁-C₄, halogénoalkyle en C₁-C₄, alcoxy en C₁-C₄ ou halogénoalcoxy en C₁-C₄,
1- ou 2-naphtyle facultativement substitué par toute association de un à trois groupes halogéno, alkyle en C₁-C₄, halogénoalkyle en C₁-C₄, alcoxy en C₁-C₄ ou halogénoalcoxy en C₁-C₄, ou
un cycle hétéroaromatique penta- ou hexagonal facultativement substitué par toute association de un à trois groupes halogéno, alkyle en C₁-C₄, halogénoalkyle en
C₁-C₄, alcoxy en C₁-C₄ ou halogénoalcoxy en C₁-C₄,
et ses isomères optiques, et ses isomères *cis* et *trans.*

2. Composé de Formule I selon la revendication 1, dans lequel
Ar est un groupe phényle facultativement substitué par toute association de un à trois groupes halogéno, alkyle en C₁-C₄, halogénoalkyle en C₁-C₄, alcoxy en C₁-C₄ ou halogénoalcoxy en C₁-C₄,
R et R₁ sont chacun indépendamment de l'hydrogène, un groupe alkyle en C₁-C₄, halogénoalkyle en C₁-C₄, cycloalkyle en C₃-C₆ ou halogénocycloalkyle en C₃-C₆, à condition qu'au moins l'un de R et R₁ soit autre chose que de l'hydrogène ; et
Ar₁ est un groupe 3-phénoxyphényle facultativement substitué par toute association de un à six groupes halogéno, alkyle en C₁-C₄, halogénoalkyle en C₁-C₄, alcoxy en C₁-C₄ ou halogénoalcoxy en C₁-C₄,
3-biphénylyle facultativement substitué par toute association de un à cinq groupes halogéno, alkyle en C₁-C₄, halogénoalkyle en C₁-C₄, alcoxy en C₁-C₄ ou halogénoalcoxy en C₁-C₄, ou
3-benzylphényle facultativement substitué par toute association de un à cinq groupes halogéno, alkyle en C₁-C₄, halogénoalkyle en C₁-C₄, alcoxy en C₁-C₄ ou halogénoalcoxy en C₁-C₄.

3. Composé de Formule I selon la revendication 1 ou la revendication 2, dans lequel
R est un groupe isopropyle ou cyclopropyle et R₁ est l'hydrogène, ou bien R et R₁ sont des groupes méthyle ;
R₂ est l'hydrogène ; et
Ar₁ est un groupe 3-phénoxyphényle facultativement substitué par toute association de un à six groupes halogéno, alkyle en C₁-C₄, halogénoalkyle en C₁-C₄, alcoxy en C₁-C₄ ou halogénoalcoxy en C₁-C₄.

4. Composé de Formule I selon l'une quelconque des revendications 1 à 3, dans lequel
R est un groupe cyclopropyle et R₁ est l'hydrogène.

5. Composé de Formule I selon la revendication 1, choisi dans la classe formée par les suivants :
1-[1-(*p*-chlorophényl)-2-fluoro-4-(4-fluoro-3-phénoxyphényl)-2-butényl]cyclopropane, (R,S)-(Z)- ;
1-[1-(*p*-chlorophényl)-2-fluoro-4-(*m*-phénoxyphényl)-2-butényl]cyclopropane, (R,S)-(Z)- ;
4-(*p*-chlorophényl)-3-fluoro-1-(4-fluoro-3-phénoxyphényl)-5-méthyl-2-hexène, (R,S)-(Z)- ;
4-(*p*-chlorophényl)-3-fluoro-5-méthyl-1-(*m*-phénoxyphényl)-2-hexène, (R,S)-(Z)- ;
4-(*p*-éthoxyphényl)-3-fluoro-1-(4-fluoro-3-phénoxyphényl)-5-méthyl-2-hexène, (R,S)-(Z)- ;
1-[1-(*p*-éthoxyphényl)-2-fluoro-4-(4-fluoro-3-phénoxyphényl)-2-butényl]cyclopropane, (R,S)-(Z)- ;
4-(*p*-éthoxyphényl)-3-fluoro-5-méthyl-1-(*m*-phénoxyphényl)-2-hexène, (R,S)-(Z)- ;
4-(*p*-éthoxyphényl)-3-fluoro-1-[*m*-(*p*-fluorophénoxy)phényl]-5-méthyl-2-hexène, (R,S)-(Z)- ;
1-{1-(*p*-chlorophényl)-2-fluoro-4-[*m*-(p-fluorophénoxy)-phényl]-2-butényl}cyclopropane, (R,S)-(Z)- ;
1-[2-fluoro-4-(4-fluoro-3-phénoxyphényl)-1-(*p*-fluorophényl)-2-butényl]cyclopropane, (R,S)-(Z)- ;
1-[1-(*p*-éthoxyphényl)-2-fluoro-4-(*m*-phénoxyphényl)-2-butényl]cyclopropane, (R,S)-(Z)- ;
1-[2-fluoro-1-(*p*-fluorophényl)-4-(*m*-phénoxyphényl)-2-butényl]cyclopropane, (R,S)-(Z)- ;
4-(*p*-chlorophényl)-3-fluoro-4-méthyl-1-(*m*-phénoxyphényl)-2-pentène, (Z)- ;
4-(*p*-chlorophényl)-3-fluoro-1-(4-fluoro-3-phénoxyphényl)-4-méthyl-2-pentène, (Z)- ;
1-(*p*-chlorophényl)-1-[1-fluoro-3-(*m*-phénoxyphényl)propényl]-cyclopropane, (Z)- ; et
1-(*p*-chlorophényl)-1-[1-fluoro-3-(4-fluoro-3-phénoxyphényl)-propényl]cyclopropane, (Z)-.

6. Procédé de lutte contre des insectes et acariens nuisibles, qui consiste à mettre en contact lesdits nuisibles ou leur source de nourriture, leur habitat ou leur site de reproduction avec une quantité à effet pesticide d'un composé de Formule I tel que revendiqué dans l'une quelconque des revendications 1 à 5.

7. Procédé pour la protection de plantes en croissance contre une attaque ou une infestation par des insectes ou acariens nuisibles, qui consiste à appliquer au feuillage des plantes, ou à la terre ou l'eau dans laquelle elles poussent, une quantité à effet pesticide d'un composé de Formule I tel que revendiqué dans l'une quelconque des revendications 1 à 5.

8. Procédé selon la revendication 7, dans lequel le composé de Formule I est appliqué aux plantes ou à la terre ou l'eau dans laquelle elles poussent, à une dose d'environ 0,1 kg/ha à 4,0 kg/ha.

9. Composition pour la lutte contre des insectes ou acariens nuisibles, qui comprend un support agronomiquement acceptable et une quantité à effet pesticide d'un composé de Formule I tel que revendiqué dans l'une quelconque des revendications 1 à 5.

10. Procédé pour la préparation d'un composé de Formule I tel que défini la revendication 1, qui comprend l'une des opérations suivantes :
a) réaction d'un 4-aryl-1-bromo-3-fluoro-2-butène de formule IX 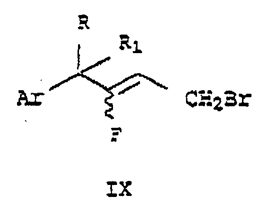 où Ar, R et R₁ sont tels que définis dans la revendication 1, avec une base ou un acide boronique ayant la formule structurale (HO)₂BAr₁, où Ar₁ est tel que défini dans la revendication 1, pour produire un composé de Formule I dans lequel R₂ est H, ou
b) réaction d'un diène de formule XVII 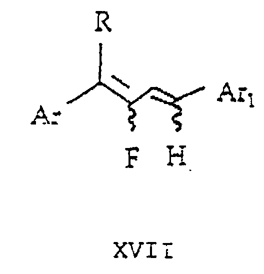 où Ar, R et Ar₁ sont tels que définis dans la revendication 1, avec du magnésium en présence d'un solvant protique pour produire le composé de Formule I correspondant dans lequel R₁ et R₂ sont tous deux H, ou
c) halogénation d'un composé de Formule I où R₂ est H avec un agent de chloration ou un agent de bromation pour produire le composé de Formule I correspondant dans lequel R₂ est Cl ou Br, ou
d) réaction d'un composé de Formule I dans lequel R₂ est Cl ou Br avec du cyanure de sodium pour produire le composé de Formule I correspondant dans lequel R₂ est CN, ou
e) réaction d'un composé de Formule I dans lequel R₂ est Cl ou Br avec de l'hydroxyde de sodium pour produire le composé de Formule I correspondant dans lequel R₂ est OH, ou
f) réaction d'un composé de Formule I dans lequel R₂ est Cl ou Br avec un composé de formule NaOR₄, où R₄ est un groupe alkyle en C₁-C₄, pour produire le composé de Formule I correspondant dans lequel R₂ est un groupe alcoxy en C₁-C₄, ou
g) réaction d'un composé de Formule I dans lequel R₂ est OH avec un halogénure d'alkyle en C₁-C₄ pour produire le composé de Formule I correspondant dans lequel R₂ est un groupe alcoxy en C₁-C₄, ou
h) réaction d'un composé de formule Br-Ar₁, où Ar₁ est tel que défini dans la revendication 1, avec un composé de la formule suivante : 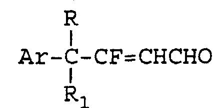 où Ar, R et R₁ sont tels que définis dans la revendication 1, pour produire un composé de Formule I dans lequel R₂ est OH, ou
i) réaction d'un composé de Formule I dans lequel R₂ est OH avec un chlorure de thionyle ou bromure de thionyle pour produire le composé de Formule I correspondant dans lequel R₂ est Cl ou Br.

11. Procédé pour la préparation d'un composé de Formule I tel que revendiqué dans l'une quelconque des revendications 1 à 5, lequel procédé comprend la réaction d'un 4-aryl-3-fluoro-2-butène-1-ol de Formule VIII 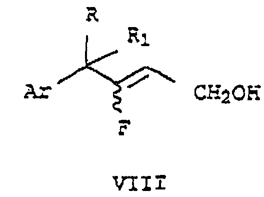 où Ar, R et R₁ sont tels que définis dans la revendication 1, avec un agent de bromation pour former un 4-aryl-1-bromo-3-fluoro-2-butène de Formule XI 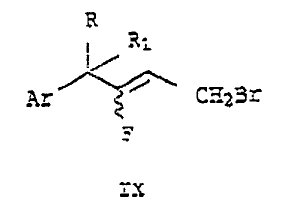 et la réaction du 4-aryl-1-bromo-3-fluoro-2-butène avec 0,0025 à 0,1 équivalent molaire d'un catalyseur au palladium, au moins 2 équivalents molaires d'une base et un acide boronique ayant la formule structurale
(HO)₂BAr₁
où Ar₁ est tel que défini dans la revendication 1.

12. Procédé pour la préparation d'un composé de Formule I tel que revendiqué dans l'une quelconque des revendications 1 à 5, lequel procédé comprend la réaction d'un 3-aryl-2-fluoro-2-propénal de Formule XV 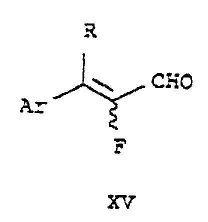 où Ar et R sont tels que définis dans la revendication 1, avec un ylure de Formule XVI 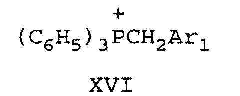 où Ar₁ est tel que défini dans la revendication 1, en présence d'une base pour former un diène ayant la formule structurale 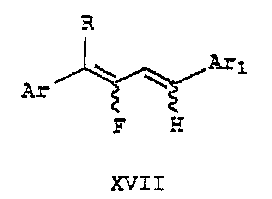 où Ar, R et Ar₁ sont tels que définis dans la revendication 1, et la réaction du diène avec du magnésium en présence d'un solvant protique.

13. Composé de Formule XXX 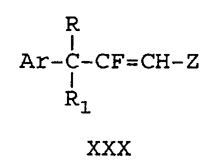 où Ar, R et R₁ sont tels que définis dans la revendication 1 et Z est un groupe cyano, CHO, CH₂OH ou CH₂Br, et ses isomères optiques, et ses isomères *cis* et *trans.*

14. Composé de Formule XXX selon la revendication 13, dans lequel
Ar est un groupe phényle facultativement substitué par toute association d'un à trois groupes halogéno, alkyle en C₁-C₄, halogénoalkyle en C₁-C₄, alcoxy en C₁-C₄ ou halogénoalcoxy en C₁-C₄.

15. Composé de Formule XXX selon la revendication 13 ou la revendication 14, dans lequel
R et R₁ sont des groupes méthyle.

16. Procédé pour la préparation d'un composé de Formule XXX tel que défini dans l'une quelconque des revendications 13 à 15, qui comprend l'une des opérations suivantes :
a) réaction d'un composé de Formule V 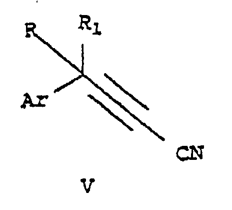 où Ar, R et R₁ sont tels que définis dans la revendication 1, avec du fluorure de césium, de l'hydrogénofluorure de potassium et de l'eau dans du N,N-diméthylformamide pour produire un composé de Formule XXX dans lequel Z = CN, ou
b) réduction sélective d'un composé de Formule XXX dans lequel Z = CN pour produire le composé de Formule XXX correspondant dans lequel Z = CHO, ou
c) réduction d'un composé de formule XXX dans lequel Z = CHO pour produire le composé de Formule XXX correspondant dans lequel Z = CH₂OH, ou
d) réaction d'un composé de Formule XXX dans lequel Z = CH₂OH avec un agent de bromation pour produire le composé de Formule XXX correspondant dans lequel Z = CH₂Br.

17. Composé de Formule XXXI 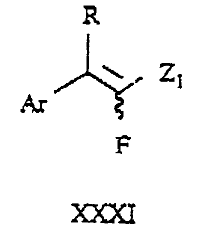 où Ar et R sont tels que définis dans la revendication 1 et
Z₁ est un groupe (alcoxy en C₁-C₄)carbonyle, CH₂OH, CHO, CH₂Cl ou CH=CHAr₁ ; et Ar₁ est tel que défini dans la revendication 1,
et ses isomères optiques et ses isomères *cis* et *trans.*

18. Composé de Formule XXXI selon la revendication 17, dans lequel
Ar est un groupe phényle facultativement substitué par toute association de un à trois groupes halogéno, alkyle en C₁-C₄, halogénoalkyle en C₁-C₄, alcoxy en C₁-C₄ ou halogénoalcoxy en C₁-C₄ ; et
Ar₁ est un groupe 3-phénoxyphényle facultativement substitué par toute association de un à six groupes halogéno, alkyle en C₁-C₄, halogénoalkyle en C₁-C₄, alcoxy en C₁-C₄ ou halogénoalcoxy en C₁-C₄.

19. Composé de Formule XXXI selon la revendication 17 ou la revendication 18, dans lequel
R est un groupe isopropyle ou cyclopropyle.

20. Procédé pour la préparation d'un composé de Formule XXXI tel que défini dans l'une quelconque des revendications 17 à 19, qui comprend l'une des opérations suivantes :
a) réaction d'une cétone ou d'un aldéhyde de formule XI 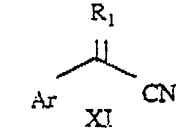 avec un ester de formule XII 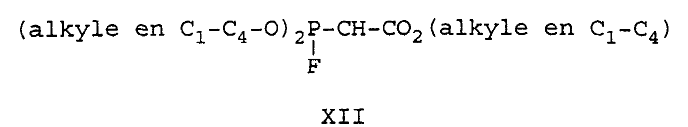 pour produire un composé de Formule XXXI dans lequel Z₁ = un groupe (alkyloxy en C₁-C₄)carbonyle, ou
b) réduction d'un composé de Formule XXXI dans lequel Z₁ = un groupe (alkyloxy en C₁-C₄) carbonyle pour produire le composé de Formule XXXI correspondant dans lequel Z₁ = CH₂OH, ou
c) oxydation d'un composé de Formule XXXI dans lequel Z₁ = CH₂OH pour produire le composé de Formule XXXI correspondant dans lequel Z₁ = CHO, ou
d) réaction d'un composé de Formule XXXI dans lequel Z₁ = CHO avec un ylure de formule XVI 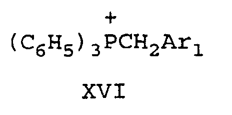 pour produire un diène de formule XVII 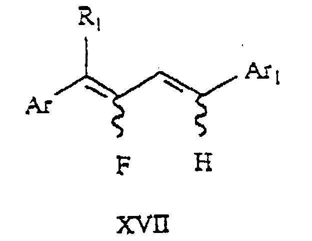 ou
e) réaction d'un composé de formule XIV 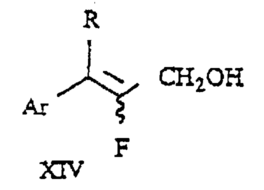 avec le chlorure de thionyle pour produire le composé de Formule XXXI correspondant dans lequel Z₁ = CH₂Cl, ou
f) réaction d'un ylure de formule XIX 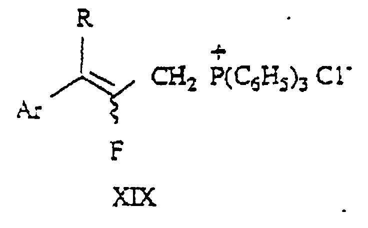 avec un aldéhyde de formule XX 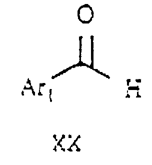 pour produire un diène de formule XVII 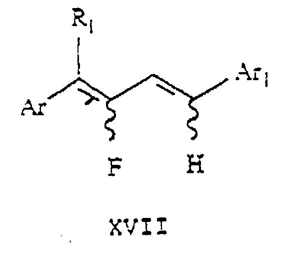 ou
g) réaction d'un composé de formule XXII 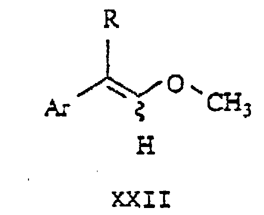 avec le dichlorofluorométhane pour produire un composé de Formule XXXI dans lequel Z₁ = CHO.
